(19) Europäisches Patentamt
European Patent Office
Office européen des brevets

(11) Veröffentlichungsnummer: **0 157 267**
**B1**

(12) # EUROPÄISCHE PATENTSCHRIFT

(45) Veröffentlichungstag der Patentschrift: **23.11.88**

(21) Anmeldenummer: **85103092.4**

(22) Anmeldetag: **18.03.85**

(51) Int. Cl.[4]: **C 07 D 311/96,** C 07 D 311/58, C 07 D 407/12, A 61 K 31/35

(54) **Substituierte Benzopyrane, Verfahren zu ihrer Herstellung sowie ihre Verwendung in Arzneimitteln.**

(30) Priorität: **31.03.84 DE 3411993**

(43) Veröffentlichungstag der Anmeldung: **09.10.85 Patentblatt 85/41**

(45) Bekanntmachung des Hinweises auf die Patenterteilung: **23.11.88 Patentblatt 88/47**

(84) Benannte Vertragsstaaten: **AT BE CH DE FR GB IT LI LU NL SE**

(56) Entgegenhaltungen:
**EP-A-0 114 374**
**GB-A-2 089 348**

(73) Patentinhaber: **BAYER AG, Konzernverwaltung RP Patentabteilung, D-5090 Leverkusen 1 Bayerwerk (DE)**

(72) Erfinder: **Kabbe, Hans- Joachim, Dr., Walter- Flex-Strasse 16, D-5090 Leverkusen 1 (DE)**
Erfinder: **Niewöhner, Ulrich, Dr., Gartenstrasse 3, D-5632 Wermelskirchen 3 (DE)**
Erfinder: **Widdig, Arno, Dr., Eifgenstrasse 8, D-5068 Odenthal (DE)**
Erfinder: **Knorr, Andreas, Dr., Pahlkestrasse 15, D-5600 Wuppertal 1 (DE)**
Erfinder: **Garthoff, Bernward, Dr., Händelstrasse 22, D-4010 Hilden (DE)**
Erfinder: **Kazda, Stanislav, Dr., Gellertweg 18, D-5600 Wuppertal 1 (DE)**

**EP 0 157 267 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft substituierte Benzopyrane, mehrere Verfahren zu ihrer Herstellung sowie ihre Verwendung in Arzneimitteln, insbesondere in kreislaufbeeinflussenden Mitteln.

Aus GB-A-2 089 348 sind bereits Benzopyrane bekannt, die sich jedoch von den erfindungsgemäßen Verbindungen sowohl strukturell als auch in ihrer biologischen Wirkung eindeutig unterscheiden. Die Benzopyrane des Standes der Technik zeigen antimykotische Wirkungen und sollen als Chemotherapeutika eingesetzt werden während die erfindungsgemäßen Verbindungen überraschenderweise eine Kreislaufwirkung besitzen. In EP-A-0 114 347 werden kreislaufwirksame Verbindungen beschrieben, die sich in ihrem Substituentenmuster von den erfindungsgemäßen Verbindungen unterscheiden. Diese Entgegenhaltung wurde jedoch erst nach dem Prioritätsdatum der vorliegenden Anmeldung (31.3.1984) veröffentlicht. Zur Beurteilung der erfinderischen Tätigkeit ist diese Veröffentlichung daher nicht heranzuziehen.

Die neuen Verbindungen können durch folgende Formel (I) wiedergegeben werden:

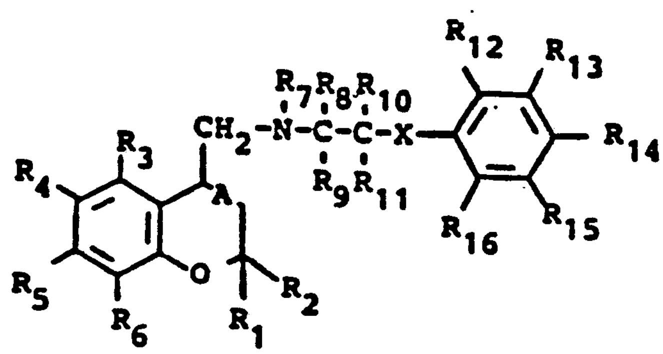

I

in der

-A- eine Einfachbindung oder eine Doppelbindung darstellt,

$R_1$ und $R_{12}$ gleich oder verschieden, für Wasserstoff, $C_1$-$C_6$-Alkyl, $C_5$-$C_7$-Cycloalkyl, Phenyl, gegebenenfalls ein- oder zweifach substituiert durch $C_1$-$C_4$-Alkyl, Halogen und/oder $C_1$-$C_4$-Alkoxy, für $C_7$-$C_9$-Aralkyl, dessen Arylrest gegebenenfalls 1- oder 2-fach substituiert ist durch $C_1$-$C_4$-Alkyl, Halogen und/oder $C_1$-$C_4$ Alkoxy stehen, oder in welcher $R_1$ und $R_2$ zusammen mit dem eingeschlossenen C-Atom des Chromanringes einen 4- bis 7-gliedrigen carbocyclischen Ring bilden;

$R_3$ bis $R_6$, gleich oder verschieden, für Wasserstoff, Hydroxy, Halogen, $C_1$-$C_6$-Alkyl, $C_5$-$C_7$-Cycloalkyl, Phenyl, gegebenenfalls 1- bis 2-fach substituiert durch $C_1$-$C_4$-Alkyl, Halogen und/oder $C_1$-$C_4$-Alkoxy oder für $C_7$-$C_9$-Aralkyl, dessen Arylrest gegebenenfalls 1- bis 2-fach substituiert ist durch $C_1$-$C_4$-Alkyl, Halogen und/oder $C_1$-$C_4$-Alkoxy,stehen;

$R_7$, $R_8$, $R_9$, $R_{10}$, $R_{11}$ gleich oder verschieden sind und für Wasserstoff oder $C_1$-$C_6$-Alkyl stehen;

X für eine Einfachbindung, für Methylen, gegebenenfalls ein- oder zweifach substituiert durch $C_1$-$C_4$-Alkyl, für Sauerstoff oder für $-NR_{17}$ steht, wobei $R_{17}$ Wasserstoff oder $C_1$-$C_4$-Alkyl darstellt oder $R_{17}$ zusammen mit $R_7$ für $C_2$-Alkylen steht;

mindestens einer und höchstens zwei der Reste $R_{12}$, $R_{13}$, $R_{14}$, $R_{15}$ und $R_{16}$, gleich und verschieden, für Nitro, Cyano oder eine der Gruppen

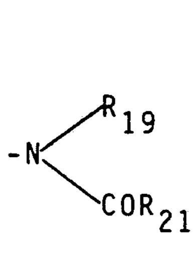 , 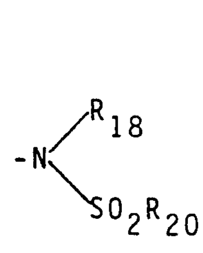 , 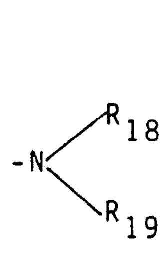 ,

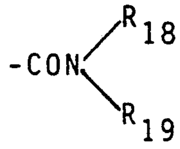 oder 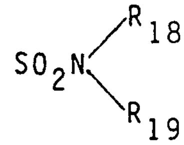 stehen, worin

$R_{18}$ und $R_{19}$ gleich oder verschieden sind und jeweils für Wasserstoff oder Alkyl mit 1 - 4 C-Atomen stehen,

welches gegebenenfalls 1- bis 3-fach substituiert ist durch Halogen,

$R_{20}$ für Alkyl mit 1-6 C-Atomen steht, welches gegebenenfalls 1- bis 3-fach substituiert ist durch Halogen, und

$R_{21}$ für Wasserstoff, Alkyl, Alkoxy oder Alkylamino mit jeweils 1-4 C-Atomen pro Alkyl und Alkoxygruppe steht, wobei der Alkylrest gegebenenfalls 1- bis 3-fach substituiert ist durch Halogen,

oder worin

$R_{18}$ gemeinsam mit $R_{19}$ oder gemeinsam mit $R_{20}$ oder gemeinsam mit $R_{21}$ einen 5- bis 6-gliedrigen heterocyclischen Ring bilden kann, der eine Alkylbrücke mit 2, 3 oder 4 Kohlenstoffatomen enthält und gegebenenfalls noch eine zusätzliche Carbonylgruppe enthält,

während die übrigen Reste $R_{12}$ bis $R_{16}$ gleich oder verschieden sind und jeweils für Wasserstoff, Hydroxy, Chlor, Fluor, Alkyl mit 1 bis 4 C-Atomen, Alkoxy mit 1 bis 3 C-Atomen, Aralkyloxy mit bis zu 8 C-Atomen, oder Trifluormethyl stehen,

und überdies

$R_{12}$ und $R_{13}$ oder $R_{13}$ und $R_{14}$ gemeinsam für eine Alkylendioxygruppe mit 1 oder 2 C-Atomen oder die Gruppe $-CH=CH-CH=CH-$ stehen können; sowie ihre pharmazeutisch unbedenklichen Additionssalze.

Als Alkyl in den Substituenten $R_1$-$R_{21}$ kommen vorzugsweise in Betracht geradkettige oder verzweigte $C_1$-$C_6$-Alkylreste, falls im Text nicht anders angegeben.

Als Alkylreste seien beispielhaft genannt:

Methyl, Ethyl, Propyl, Isopropyl, Butyl, Isobutyl, tert.-Butyl, Hexyl, 2-Hexyl, 1,1-Dimethylpentyl, 1,1-Dimethylhexyl.

Als Cycloalkylreste in den Resten $R_1$, $R_2$, $R_3$, $R_4$, $R_5$, $R_6$, $R_{12}$ bis $R_{16}$ kommen vorzugsweise solche mit 3 bis 6 Kohlenstoffatomen wie beispielsweise Cyclopropyl, Cyclobutyl, Cyclopentyl, Cyclohexyl, insbesondere Cyclopentyl und Cyclohexyl in Frage.

Als gegebenenfalls substituiertes Alkyl in den Resten $R_{12}$-$R_{21}$ stehen vorzugsweise Alkylgruppen, die 1-, 2- oder 3-fach substituiert sind durch Hydroxy, Halogen, insbesondere Fluor oder Chlor, Cyano, Nitro, Alkoxy mit 1 bis 4 C-Atomen oder Trifluormethoxy.

Als gegebenenfalls substituierte Aralkylreste in den Resten $R_1$, $R_2$ und $R_3$ bis $R_6$ seien beispielhaft genannt: Benzyl, Phenylethyl, Phenylpropyl, Phenylbutyl, Naphthylmethyl, bevorzugt Benzyl.

Als Alkoxy in den Resten $R_3$ bis $R_6$ und $R_{12}$ bis $R_{21}$ seien beispielhaft Methoxy, Ethoxy, n- und i-Propoxy und n-, i- und t-Butoxy genannt. Als Aryloxygruppen $R_3$ bis $R_6$ und $R_{12}$ bis $R_{16}$ seien Phenoxy oder Naphthoxy genannt.

Als Aralkoxy in den Resten $R_3$ bis $R_6$ seien Benzyloxy, Phenyloxy, Phenylpropoxy, Phenylisopropoxy, Phenylbutoxy und Phenylisobutoxy genannt.

Als Halogene in den Resten $R_3$ bis $R_6$ und $R_{12}$ bis $R_{21}$ seien bevorzugt Fluor Brom und Chlor genannt.

Falls die Reste $R_1$ und $R_2$ zusammen mit dem durch sie eingeschlossenen Kohlenstoffatom einen carbocyclischen Ring bilden, so kommen bevorzugt 3- bis 7-gliedrige Ringe in Frage. Als carbocyclische Resten seien beispielsweise genannt: Cyclopropan, Cyclobutan, Cyclopentan, Cyclohexan, Cycloheptan.

Als Substituenten der Aryl-, Aralkyl-, Aryloxy- und Aralkyloxy-Reste $R_1$ bis $R_6$ kommen Substituenten in Frage, die unter den Reaktionsbedingungen nicht verändert werden. Beispielsweise seien die Halogene wie Fluor, Chlor, Brom und Iod, die $C_1$-$C_6$-Alkylgruppe, die $C_1$-$C_6$-Alkoxygruppe und die Trifluormethylgruppe genannt.

Als Säuren zur Herstellung der Salze seien beispielsweise genannt: Schwefelsäure, Salzsäure, organische Carbonsäuren wie Äpfelsäure, Citronensäure, Fumarsäure, Maleinsäure, Bernsteinsäure, Essigsäure oder organische Sulfonsäuren wie Naphthalin-1,5-disulfonsäure.

Die Salze der Verbindungen der Formel (I) können in einfacher Weise nach üblichen Salzbildungsmethoden, z. B. durch Lösen der Base und Hinzufügen der Säure erhalten werden und in bekannter Weise, z. B. durch Abfiltrieren, isoliert und gegebenenfalls gereinigt werden.

Bevorzugt sind Verbindungen der Formel (I), in welcher

$R_1$ und $R_2$ gleich oder verschieden sind und für Wasserstoff, $C_1$-$C_4$-Alkyl stehen oder zusammen mit dem von ihnen eingeschlossenen Kohlenstoffatom einen carbocyclischen $C_5$ oder $C_6$ Ring bilden,

$R_3$ bis $R_6$ gleich oder verschieden sind und Wasserstoff, Hydroxy, $C_1$-$C_4$-Alkyl, $C_1$-$C_4$-Alkoxy oder Chlor bedeuten,

$R_7$ bis $R_{11}$ gleich oder verschieden sind und Wasserstoff oder $C_1$-$C_4$-Alkyl darstellen,

X für eine Einfachbindung, für Sauerstoff, für Methylen oder für $-NR_{17}$ steht, wobei $R_{17}$ Wasserstoff oder $C_1$-$C_3$-Alkyl bedeutet, oder wobei $R_{17}$ zusammen mit $R_7$ einen Ethylenringschluß bildet, und

$R_{12}$ bis $R_{16}$ gleich oder verschieden sind und Wasserstoff, Chlor, Cyclohexyl, $C_1$-$C_4$-Alkyl, $C_1$-$C_3$-Alkoxy, Aralkoxy mit 1-8 C-Atomen, Trifluormethyl bedeuten, wobei $R_{12}$ und $R_{13}$ oder $R_{13}$ und $R_{14}$ gegebenenfalls zusammen jeweils eine Methylendioxygruppe oder eine $-CH=CH-CH=CH-$Gruppe bilden,

und wobei mindestens eine und höchstens zwei Substituenten aus der Gruppe $R_{12}$ - $R_{16}$ gleich und verschieden sind und jeweils für Nitro, Cyano, die Gruppe

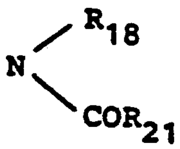, 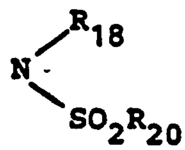, 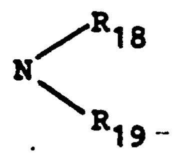, 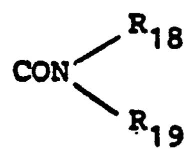,

oder 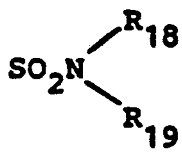 stehen, wobei $R_{18}$ und $R_{19}$

stehen, wobei

$R_{18}$ und $R_{19}$ gleich oder verschieden sind und jeweils für Wasserstoff oder gegebenenfalls durch Fluor oder Chlor substituiertes Alkyl mit 1-4 C-Atomen stehen und wobei $R_{20}$ für gegebenenfalls durch Fluor oder Chlor substituiertes Alkyl mit 1 - 4 C-Atomen steht und wobei $R_{21}$ für Wasserstoff, gegebenenfalls durch Fluor oder Chlor substituiertes Alkyl mit 1-4 C-Atomen, Alkoxy oder Alkylamino mit jeweils 1-2 C-Atomen steht.

Als neue Verbindungen der allgemeinen Formel (I) seien beispielhaft genannt:

4-{N-[4-(3-Nitrophenyl)-2-butyl]-aminomethyl}-chroman
4-{N-[4-(2-Chlor-4-nitrophenyl)-2-butyl]-aminomethyl}-2,2-dimethylchromen
4-{N-[4-(3,4-Methylendioxy-5-nitrophenyl)-2-butyl]-aminomethyl}-7-hydroxy-2,2-diisopropylchroman
4-{N-[4-(4-Methyl-5-nitrophenyl)-2-butyl]-aminomethyl}-2-spirocyclohexachroman
4-{N-[4-(3-Trifluormethyl-5-nitrophenyl)-2-butyl]-aminomethyl}-6,7-dimethyl-2-spirocyclopentachroman
4-{N-[4-(3,5-Dinitrophenyl)-2-butyl]-aminomethyl}-7-chlor-2-benzylchromen
4-{N-[4-(3-Methoxy-4-hydroxy-5-nitrophenyl)-2-butyl]-aminomethyl}-5-methoxy-2-spirocyclopentachroman
4-{N-[4-(3-N-Methylaminophenyl)-2-butyl]-aminomethyl}-2-spirocyclopentachroman
4-{N-[4-(3-Methoxy-4-aminophenyl)-2-butyl]-aminomethyl-2-spirocyclopentachroman
4-{N-[4-(3-Pyrrolidinophenyl)-2-butyl]-aminomethyl}-2-spirocyclopentachroman
4-{N-[4-(3-Methylsulfonylaminophenyl)-2-butyl]-aminomethyl}-chroman
4-{N-[4-(2-Chlor-4-methylsulfonylaminophenyl)-2-butyl]-aminomethyl}-2,2-dimethylchromen
4-{N-[4-(3,4-Methylendioxy-5-methylsulfonylaminophenyl)-2-butyl]-aminomethyl}-7-hydroxy-2,2-diisopropylchroman
4-{N-[4-(4-Methyl-5-methylsulfonylaminophenyl)-2-butyl]-aminomethyl}-2-spirocyclohexachroman
4-{N-[4-(3-Trifluormethyl-5-methylsulfonylaminophenyl)-2-butyl]-aminomethyl}-6,7-dimethyl-2-spirocyclopentachromen
4-{[4-(3,5-Bis-methylsulfonylaminophenyl)-2-butyl]-aminomethyl}-6-chlor-2-benzylchromen
4-{[4-(3-Methoxy-4-hydroxy-5-methylsulfonylaminophenyl)-2-butyl]-aminomethyl}-2-spirocyclopentachroman
4-{N-[4-(3-Acetylaminophenyl)-2-butyl]-aminomethyl}-chroman
4-{N-[4-(2-Chlor-4-acetylaminophenyl)-2-butyl]-aminomethyl}-2,2-dimethylchromen
4-{N-[4-(3,4-Methylendioxy-5-acetylaminophenyl)-2-butyl]-aminomethyl}-7-hydroxy-2,2-diisopropylchroman
4-{N-[4-(4-Methyl-5-acetylaminophenyl)-2-butyl]-aminomethyl}-2-spirocyclohexachroman
4-{N-[4-(3-Trifluormethyl-5-acetylaminophenyl)-2-butyl]-aminomethyl}-6,7-dimethyl-2-spirocyclopentachromen
4-{N-[4-(3,5-Dinitrophenyl)-2-butyl]-aminomethyl}-7-chlor-2-benzylchromen
4-{-N-[4-(3-Methoxy-4-hydroxy-5-acetylaminophenyl)-2-butyl]-aminomethyl}-5-methoxy-2-spirocyclopentachroman
4-{-N-[4-(4-Cyanophenyl)-2-butyl]-aminomethyl}-2-spirocyclopentachroman
4-{N-[4-(4-Methyl-5-cyanophenyl)-2-butyl]-aminomethyl-2-spirocyclopentachroman
4-{N-[4-(4-Methoxy-5-cyanophenyl)-2-butyl]-aminomethyl}-2-spirocyclopentachroman
4-{N-[1-(3-Methoxy-4-methylsulfonylaminophenoxy)-2-propyl]-aminomethyl}-2-spirocyclopentachromen
4-{N-[1-(3-Aminophenoxy)-2-propyl]-aminomethyl}-2,2-dimethylchromen
4-{N-[1-(3-N-Methylmethylsulfonylaminophenoxy)-2-propyl]-aminomethyl}-7-chlor-2-spirocyclohexachroman
4-{N-[1-(3-N-Acetylaminophenoxy)-2-propyl]-6,7-dimethyl-}-2-spirocyclopentachroman

Die Erfindung betrifft weiterhin verschiedene Verfahren zur Herstellung der Verbindungen der Formel (I). Man setzt entweder

A) Chroman-4-carbaldehyde der Formel (II)

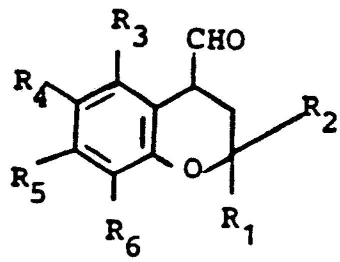

(II)

in welcher
$R_1$, $R_2$, $R_3$, $R_4$, $R_5$ und $R_6$ die oben angegebene Bedeutung haben,
mit aminen der Formel (III)

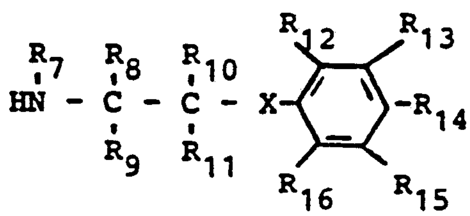

(III)

in welcher
$R_7$, $R_8$, $R_9$, $R_{10}$, $R_{11}$, $R_{12}$, $R_{13}$, $R_{14}$, $R_{15}$, $R_{16}$, $R_{17}$ und X die oben angegebene Bedeutung haben,
in Gegenwart von reduzierenden Agentien um,
oder
B) Amine der Formel (IV)

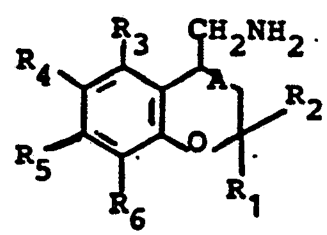

(IV)

in welcher
A, $R_1$, $R_2$, $R_3$, $R_4$, $R_5$ und $R_6$ die oben angegebene Bedeutung haben,
mit Carbonylverbindungen der Formel (V)

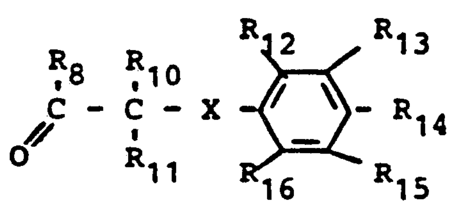

(V)

in welcher
$R_8$ bis $R_{16}$ die oben angegebene Bedeutung haben, mit der Maßgabe, daß X nicht für $NR_{17}$ steht,
in Gegenwart von reduzierenden Agentien um,
oder

C) Verbindungen der Formel (VI)

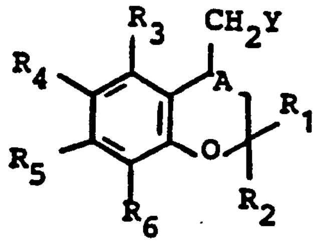

(VI)

in welcher

A, $R_1$, $R_2$, $R_3$, $R_4$, $R_5$ und $R_6$ die oben angegebene Bedeutung haben, und

Y für nucleophil austauschbare Gruppen, wie Alkyl- oder Aryl substituierte Sulfonyloxygruppen, Brom oder Chlor steht,

mit den Aminen der Formel (III) in Gegenwart eines säurebindenden Mittels um.

Als reduzierende Agentien, wie sie in den Verfahrensvarianten A und B eingesetzt werden können, seien beispielhaft komplexe Metallhydride genannt. Bevorzugt sind Alkaliborhydride, Alkalicyanoborhydride und/oder Alkalialanate, insbesondere Natrium- oder Lithiumverbindungen. Namentlich genannt seien Natriumborhydrid, Natriumcyanoborhydrid oder Lithiumalanat. Ebenso kann auch katalytisch erregter Wasserstoff bei erhöhten Drucken und Temperaturen eingesetzt werden.

Die reduzierenden Agentien können in äquivalenten Mengen bis zum Überschuß von 100 %, bevorzugt von äquivalenten Mengen bis zum Überschuß von 20 %, bezogen auf die eingesetzte Carbonylverbindung, eingesetzt werden.

Die in der Herstellungsvariante C eingesetzten säurebindenden Mittel sind bekannte Basen. Beispielhaft seien genannt: Erdalkali- oder Alkalihydroxide wie Natrium- und/oder Kaliumhydroxid, Erdalkali- oder Alkalicarbonate wie Natriumbicarbonat oder Kaliumcarbonat, organische Stickstoffbasen wie Triethylamin, Tributylamin oder Benzyltrimethylammoniumhydroxid.

Diese säurebindenden Mittel können in äquivalenten Mengen bis zum Überschuß von 100 %, bevorzugt in äquivalenten Mengen bis zum Überschuß von 20 %, bezogen auf die eingesetzte Halogenverbindung, eingesetzt werden.

Die erfindungsgemäßen Reaktionen werden in Lösungsmitteln durchgeführt. Als Lösungsmittel kommen alle für die jeweilige Reaktion inerten Lösungsmittel in Frage, vorzugsweise seien genannt:

Alkohole wie Methanol, Ethanol, Isopropanol oder tert.-Butanol, Ether wie Diethylether, Diisopropylether, Tetrahydrofuran oder Dioxan, Kohlenwasserstoffe wie Hexan, Cyclohexan, Benzol oder Toluol, Chlorkohlenwasserstoffe wie Methylenchlorid, Chloroform, Tetrachlorkohlenstoff oder Chlorbenzol, ferner Acetonitril, Dimethylformamid, Dimethylsulfoxid oder Mischungen solcher Lösungsmittel.

Die Reaktionstemperaturen können in einem größeren Bereich variiert werden. Im allgemeinen arbeitet man zwischen -50 und 150°C, vorzugsweise von -10 bis 120°C.

Bei der Durchführung der erfindungsgemäßen Verfahren setzt man bevorzugt bezogen auf 1 Mol der Chroman- oder Chromenverbindung 0,5 bis 2 Mole des Amins der Formel (III) oder der Carbonylverbindung der Formel (V) um.

Besonders bevorzugt beträgt das Molverhältnis der Reaktionspartner 1 : 1. Benutzt man einen Überschuß, so setzt man bevorzugt einen Überschuß an Amin der Formel (III) oder an Carbonylverbindung der Formel (V) ein.

Die Reaktionsprodukte können durch Destillation, Auskristallisieren, Einengen und Umlösen oder chromatographische Trennung gewonnen werden.

Die Herstellung der Chroman-4-aldehyde der Formel (II) erfolgt durch Hydroformylierung aus 2H-Chromenen gemäß folgender Gleichung:

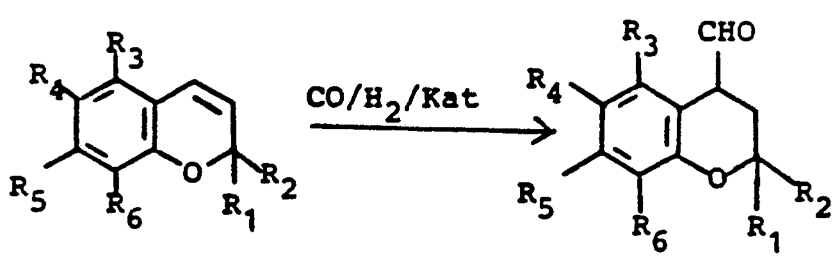


Das Verfahren ist dadurch gekennzeichnet, daß die 2H-Chromene mit Kohlenmonoxid und Wasserstoff in Gegenwart von Metallkatalysatoren aus der 8. Nebengruppe des Periodensystems bei Temperaturen von 80 bis 250°C und Drucken von 20 - 1000 bar umgesetzt werden.

Als Beispiele für die Chroman-4-carbaldehyde (II) seien genannt:

4-Formylchroman, 4-Formyl-2-methylchroman, 4-Formyl-2,2-dimethylchroman, 4-Formyl-2-propylchroman, 4-Formyl-2-isopropylchroman, 4-Formyl-2,2-diethylchroman, 4-Formyl-2-methyl-2-propylchroman, 4-Formyl-2-hexylchroman, 4-Formyl-2-cyclopentylchroman, 4-Formyl-2-cyclohexylchroman, 4-Formyl-2-spirocyclopentachroman, 4-Formyl-2-spirocyclohexachroman, 4-Formyl-6-methyl-2-spirocyclopentachroman, 4-Formyl-7-methyl-2-spirocyclopentachroman, 4-Formyl-6,8-dimethyl-2-spirocyclopentachroman, 4-Formyl-6-chlor-2-spirocyclopentachroman, 4-Formyl-6-methoxy-2-spirocyclopentachroman, 4-Formyl-7-methoxy-2-spirocyclopentachroman, 4-Formyl-7-isopropoxy-2-spirocyclopentachroman, 4-Formyl-7-phenoxy-2-spirocyclopentachroman, 4-Formyl-7-benzyloxy-2-spirocyclopentachroman, 4-Formyl-7-phenyl-2-spirocyclopentachroman, 4-Formyl-6-methyl-2,2-dimethylchroman, 4-Formyl-6-chlor-2,2-dimethylchroman, 4-Formyl-7-methoxy-2,2-dimethylchroman, 4-Formyl-6-methyl-2-spirocyclohexachroman, 4-Formyl-7-methoxy-2-spirocyclohexachroman.

Die bei der Herstellung der erfindungsgemäßen Verbindungen eingesetzten Amine der Formel (III) sind zum Teil bekannt (vgl. z. B. Arch. Pharm. 316, 193 (1983)) bzw. können nach analogen Verfahren erhalten werden. Als Beispiele für die Amine der Formel (III) seien genannt:

1-(4-Hydroxyphenyl)-2-methyl-isopropylamin, 1-(2-Methoxy-4-hydroxyphenyl)-2-methyl-isopropylamin, 1-(2,6-Dimethyl-4-hydroxyphenyl)-2-methyl-isopropylamin, 1-(2-Chlor-4-hydroxyphenyl)-2-methyl-isopropylamin, 1-(3,5-Dimethyl-4-methoxyphenyl)-2-methyl-isopropylamin, 1-(3,4,5-Trimethoxyphenyl)-2-methyl-isopropylamin, 1-(2,6-Dichlor-4-methoxyphenyl)-2-methyl-isopropylamin, 1-(3,5-Dichlor-4-methoxyphenyl)-2-methyl-isopropylamin,

2-Amino-4-(3-nitrophenyl)-butan, 2-Amino-4-(3-methylsulfonylaminophenyl)-butan, 2-Amino-4-(4-methylsulfonylaminophenyl)-butan, 2-Amino-4-(3-acetaminophenyl)-butan, 2-Amino-4-(2-nitrophenyl)-butan, 2-Amino-4-(3-N,N-dimethylaminosulfonylphenyl)-butan, 2-Amino-4-(3-N-methylcarbamoylphenyl)-butan, 2-Amino-4-(3,4-dimethoxy-5-methylsulfonylaminophenyl)-butan.

Die bei der Herstellung der erfindungsgemäßen Verbindungen eingesetzten Amine der Formel (IV) sind teilweise bekannt (J. med. Chem. 1982, 393), oder können nach analogen Verfahrensweisen dargestellt werden. Beispielhaft seien folgende Verfahrenswege schematisch angegeben, ausgehend von Chroman-4-onen:

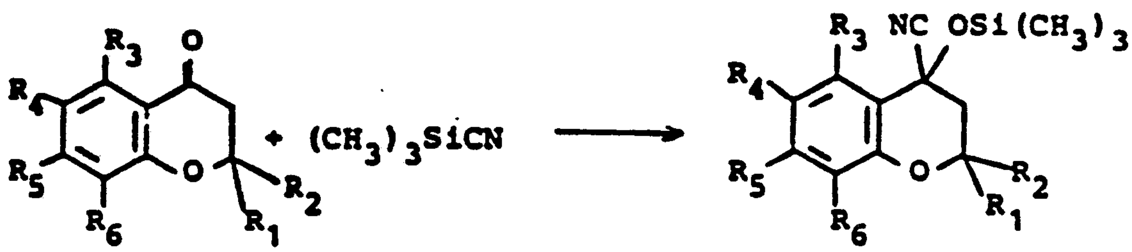


Die hierbei entstehenden 4-Cyan-4-trimethylsilyloxychromane können mit Lithiumaluminiumhydrid zu 4-Aminomethyl-4-hydroxychromanen hydriert werden, wie in folgendem Schema angegeben:

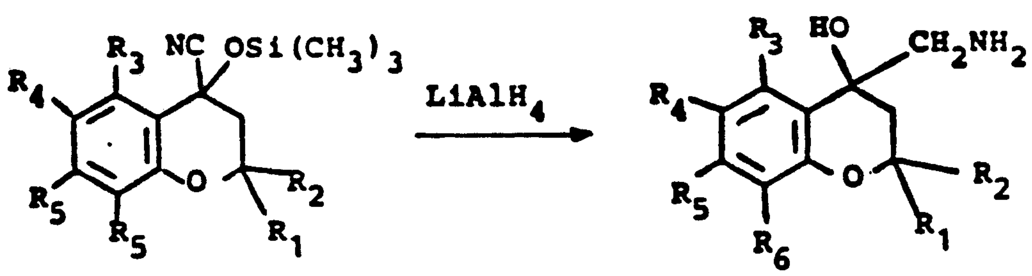

Die 4-Aminomethyl-4-hydroxy-chromane können durch wasserabspaltende Mittel in die 4-Aminomethyl-2H-chromene der Formel (IV), wie im folgenden Schema gezeigt, übergeführt werden.

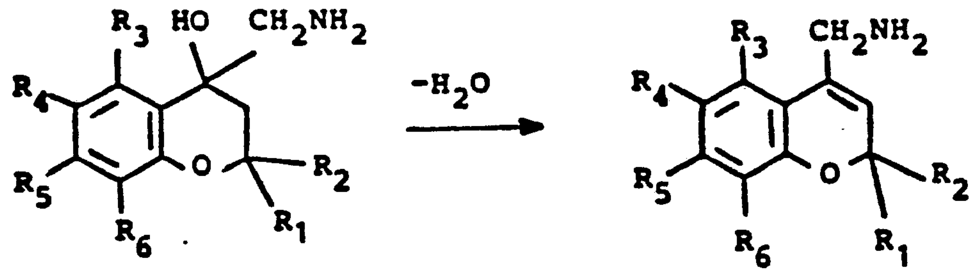


Aus den Aminomethylchromenen lassen sich die Aminomethylchromane durch Hydrierung wie in folgendem Schema gewinnen:

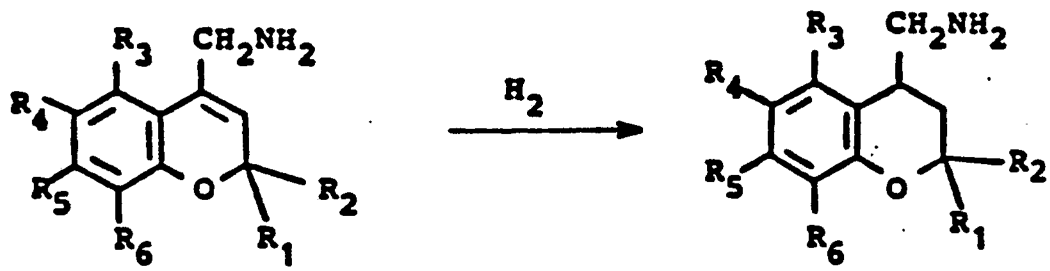


Man kann aber auch aus den 4-Cyan-4-trimethylsilyloxychromanen mit Hilfe von Phosphoroxychlorid Trimethylsilanol abspalten und so 4-Cyan-2H-chromene gewinnen, wie im folgenden Schema angegeben (analog zu einer Verfahrensweise aus Chemistry Letters 1979, 1427):

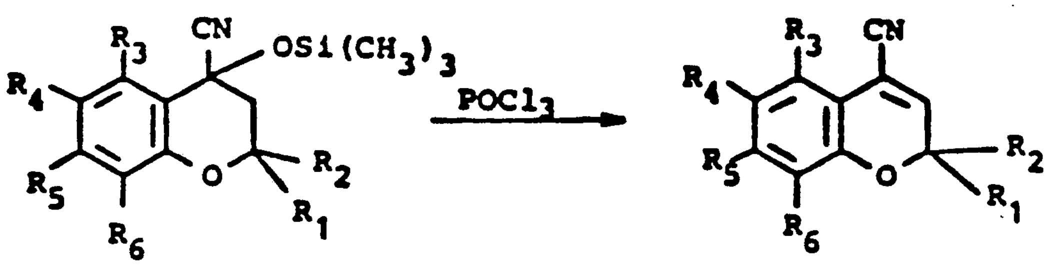


Die 4-Cyan-2H-chromene können dann über die 4-Cyanchromane direkt oder stufenweise zu den 4-Aminomethylchromanen der Formel (IV) hydriert werden, wie im folgenden Schema dargestellt:

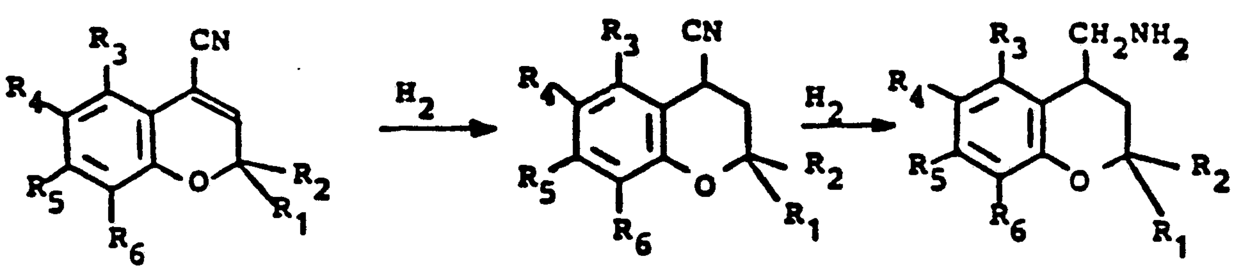


Als Beispiele für die 4-Aminomethylchromene-2H (IV) seien genannt:

4-Aminomethyl-2H-chromen,
4-Aminomethyl-2-methyl-2H-chromen,
4-Aminomethyl-2,2-dimethylchromen,
4-Aminomethyl-2-propyl-2H-chromen,
4-Aminomethyl-2-isopropyl-2H-chromen,
4-Aminomethyl-2,2-diethylchromen,

4-Aminomethyl-2-methyl-2-propylchromen,
4-Aminomethyl-2-hexyl-2H-chromen,
4-Aminomethyl-2-cyclopentyl-2H-chromen,
4-Aminomethyl-2-cyclohexyl-2H-chromen,
4-Aminomethyl-2-spirocyclopentachromen,
4-Aminomethyl-2-spirocyclohexachromen,
4-Aminomethyl-6-methyl-2-spirocyclopentachromen,
4-Aminomethyl-7-methyl-2-spirocyclopentachromen,
4-Aminomethyl-6,8-dimethyl-2-spirocyclopentachromen,
4-Aminomethyl-6-chlor-2-spirocyclopentachromen,
4-Aminomethyl-6-methoxy-2-spirocyclopentachromen,
4-Aminomethyl-7-methoxy-2-spirocyclopentachromen,
4-Aminomethyl-7-isopropoxy-2-spirocyclopentachromen,
4-Aminomethyl-7-phenoxy-2-spirocyclopentachromen,
4-Aminomethyl-7-benzyloxy-2-spirocyclopentachromen,
4-Aminomethyl-7-phenyl-2-spirocyclopentachromen,
4-Aminomethyl-6-methyl-2-spirocyclohexachromen,
4-Aminomethyl-6-chlor-2-spirocyclohexachromen,
4-Aminomethyl-7-methoxy-2-spirocyclohexachromen,
4-Aminomethyl-6-methyl-2,2-dimethylchromen,
4-Aminomethyl-7-methoxy-2,2-dimethylchromen.

Die bei der Herstellung der erfindungsgemäßen Verbindungen eingesetzten Amine der Formel (IV) sind teils bekannt (J. med. Chem. 1982, 393) oder können nach analogen Verfahrensweisen, beispielsweise wie oben beschrieben, gewonnen werden.
Als Beispiele für die 4-Aminomethylchromane (IV) seien genannt:

4-Aminomethylchroman,
4-Aminomethyl-2-methylchroman,
4-Aminomethyl-2,2-dimethylchroman,
4-Aminomethyl-2-propylchroman,
4-Aminomethyl-2-isopropylchroman,
4-Aminomethyl-2,2-diethylchroman,
4-Aminomethyl-2-methyl-2-propylchroman,
4-Aminomethyl-2-hexylchroman,
4-Aminomethyl-2-cyclopentylchroman,
4-Aminomethyl-2-cyclohexylchroman,
4-Aminomethyl-2-spirocyclopentachroman,
4-Aminomethyl-2-spirocyclohexachroman,
4-Aminomethyl-6-methyl-2-spirocyclopentachroman,
4-Aminomethyl-7-methyl-2-spirocyclopentachroman,
4-Aminomethyl-6,8-dimethyl-2-spirocyclopentachroman,
4-Aminomethyl-6-chlor-2-spirocyclopentachroman,
4-Aminomethyl-6-methoxy-2-spirocyclopentachroman,
4-Aminomethyl-7-methoxy-2-spirocyclopentachroman,
4-Aminomethyl-8-isopropoxy-2-spirocyclopentachroman,
4-Aminomethyl-7-phenoxy-2-spirocyclopentachroman,
4-Aminomethyl-7-benzyloxy-2-spirocyclopentachroman,
4-Aminomethyl-7-phenyl-2-spirocyclopentachroman,
4-Aminomethyl-6-methyl-2-spirocyclohexachroman,
4-Aminomethyl-6-chlor-2-spirocyclohexachroman,
4-Aminomethyl-7-methoxy-2-spirocyclohexachroman,
4-Aminomethyl-6-methyl-2,2-dimethylchroman,
4-Aminomethyl-7-methoxy-2,2-dimethylchroman.

Die bei der Herstellung der erfindungsgemäßen Verbindungen verwendeten Carbonylverbindungen der Formel (V) sind bekannt (vgl. z. B. Beilsteins Handbuch der organischen Chemie 6, 151, II 152; 7, 292, 303, 304, 314, I 154, 161, 162, 167, II 226, 233, 236, 243) oder können nach analogen Verfahren hergestellt werden.
Als Beispiele für die Carbonylverbindungen (V) seien genannt:

4-(3-Carbamoylphenyl)-butanon-2; 4-(4-Carbamoylphenyl)-butanon-2; 4-(2-Carbamoylphenyl)-butanon-2; 4-(2-Chlor-5-carbamoylphenyl)-butanon-2; 4-(4,5-Dimethoxy-3-carbamoylphenyl)-butanon-2; 4-(3-Methoxy-5-carbamoylphenyl)-butanon-2; 4-(3-Methyl-5-carbamoylphenyl)-butanon-2; 4-(3-Trifluormethyl-5-carbamoylphenyl)-butanon-2; 4-(3-Methoxy-4-hydroxy-5-carbamoylphenyl)-butanon-2; 4-(4-Methoxy-5-carbamoylphenyl)-butanon-2; 4-(2-Methyl-4-carbamoylphenyl)-butanon-2; 4-(2,4-Dichlor-5-carbamoylphenyl)-butanon-2; 4-(3,4-Methylendioxy-5-carbamoylphenyl)-butanon-2;

4-(3-Aminosulfonylphenyl)-butanon-2; 4-(4-Aminosulfonylphenyl)-butanon-2; 4-(2-Aminosulfonylphenyl)-butanon-2; 4-(2-Chlor-5-aminosulfonylphenyl)-butanon-2; 4-(4,5-Dimethoxy-3-aminosulfonylphenyl)-butanon-2; 4-(3-Methoxy-5-aminosulfonylphenyl)-butanon-2; 4-(3-Methyl-5-aminosulfonylphenyl)-butanon-2; 4-(3-Trifluormethyl-5-aminosulfonylphenyl)-butanon-2;

4-(3-Methoxy-4-hydroxy-5-aminosulfonylphenyl)-butanon-2; 4-(3-Chlor-5-aminosulfonylphenyl)-butanon-2; 4-(4-Methoxy-5-aminosulfonylphenyl)-butanon-2; 4-(2-Methyl-4-aminosulfonylphenyl)-butanon-2; 4-(2,4-Dichlor-5-aminosulfonylphenyl)-butanon-2; 4-(3,4-Methylendioxy-5-Aminosulfonylphenyl)-butanon-2;

4-(3-Aminophenyl)-butanon-2; 4-(3-N-Methylaminophenyl)-butanon-2; 4-(3-N,N-Dimethylaminophenyl)-butanon-2; 4-(3-N-Succinimidophenyl)-butanon-2; 4-(3-Ethylsulfonylaminophenyl)-butanon-2; 4-(3-n-Butylsulfonylaminophenyl)-butanon-2; 4-(3-N-Methyl-methylsulfonylaminophenyl)-butanon-2; 4-(3-Propionylaminophenyl)-butanon-2; 4-(3-N-Methyl-acetylaminophenyl)-butanon-2; 4-(4-Cyanophenyl)-butanon-2; 4-(4-Methoxy-5-cyanophenyl)-butanon-2; 4-(3-Pyrrolidinophenyl)-butanon-2; 1-(4-Nitrophenoxyl-propanon-2; 1-(3-Nitrophenoxy)-propanon-2; 1-(3-Aminophenoxy)-propanon-2; 1-(3-Methylsulfonylaminophenoxy]-propanon-2; 1-(4-N-Methyl-methylsulfonylaminophenoxy)-propanon-2; 1-(3-N-Acetylaminophenoxy)-propanon-2; 1-(3-N,N-Dimethylaminosulfonylphenoxy)-propanon-2; 1-(4-Aminosulfonylphenoxy)-propanon-2; 1-(4-Carbamoylphenoxy)-propanon-2;

4-(3-Nitrophenyl)-butanon-2; 4-(4-Nitrophenyl)-butanon-2; 4-(2-Nitrophenyl)-butanon-2; 4-(2-Chlor-4-nitrophenyl)-butanon-2; 4-(4,5-Dimethoxy-3-nitrophenyl)-butanon-2; 4-(3-Methoxy-5-nitrophenyl)-butanon-2; 4-(3-Methyl-5-nitrophenyl)-butanon-2; 4-(3-Trifluormethyl-5-nitrophenyl)-butanon-2; 4-(3,5-Dinitrophenyl)-butanon-2; 4-(3-Methoxy-4-hydroxy-5-nitrophenyl)-butanon-2; 4-(3-Chlor-5-nitrophenyl)-butanon-2; 4-(4-Methoxy-5-nitrophenyl)-butanon-2; 4-(2-Methyl-4-nitrophenyl)-butanon-2; 4-(2,4-Dichlor-5-nitrophenyl)-butanon-2; 4-(3,4-Methylendioxy-5-nitrophenyl)-butanon-2; 4-(3-Methylsulfonylaminophenyl)-butanon-2; 4-(4-Methylsulfonylaminophenyl)-butanon-2; 4-(2-Methylsulfonylaminophenyl)-butanon-2; 4-(2-Chlor-5-methylsulfonylaminophenyl)-butanon-2; 4-(4,5-Dimethoxy-3-methylsulfonylaminophenyl)-butanon-2; 4-(3-Methoxy-5-methyl-sulfonylaminophenyl)-butanon-2; 4-(3-Methyl-5-methylsulfonylaminophenyl)-butanon-2; 4-(3-Trifluormethyl-5-methylsulfonylaminophenyl)-butanon-2; 4-(3,5-bis-Methylsulfonylaminophenyl)-butanon-2; 4-(3-Methoxy-4-hydroxy-5-methylsulfonylaminophenyl)-butanon-2; 4-(3-Chlor-5-methylsulfonylaminophenyl)-butanon-2; 4-(4-Methoxy-5-methylsulfonylaminophenyl)-butanon-2; 4-(2-Methyl-4-methylsulfunylaminophenyl)-butanon-2; 4-(2,4-Dichlor-5-methylsulfonylaminophenyl)-butanon-2; 4-(3,4-Methylendioxy-5-methylsulfonylaminophenyl)-butanon-2; 4-(3-Acetylaminophenyl)-butanon-2; 4-(C-Acetylaminophenyl)-butanon-2; 4-(2-Acetylaminophenyl)-butanon-2; 4-(2-Chlor-5-acetylaminophenyl)-butanon-2; 4-(4,5-Dimethoxy-3-acetylaminophenyl)-butanon-2; 4-(3-Methoxy-5-acetylaminophenyl)-butanon-2; 4-(3-Methyl-5-acetylaminophenyl)-butanon-2; 4-(3-Trifluormethyl-5-acetylaminophenyl)-butanon-2; 4-(3,5-bis-Acetylaminophenyl)-butanon-2; 4-(3-Methoxy-4-hydroxy-5-acetylaminophenyl)-butanon-2; 4-(3-Chlor-5-acetylaminophenyl)-butanon-2; 4-(4-Methoxy-5-acetylaminophenyl)-butanon-2; 4-(2-Methyl-4-acetylaminophenyl)-butanon-2; 4-(2,4-Dichlor-5-acetylaminophenyl)-butanon-2; 4-(3,4-Methylendioxy-5-acetylaminophenyl)-butanon-2.

Die bei der Herstellung der erfindungsgemäßen Verbindungen eingesetzten Verbindungen der Formel (VI) sind zum Teil bekannt (Tetrahedron 23, 1893 (1967)), oder können auf analogem Weg oder aus 4-Methylchromenen-2H [Heterocyclic Compounds, Vol. 31, Ed. G.P. Ellis (New York 1977), p 11 ff.] durch Halogenierung mit N-Brom- oder N-Chlorsuccinimid gewonnen werden (Wohl-Ziegler-Reaktion).

Als Beispiele für die Verbindungen der Formel VI seien genannt:

4-Chlormethyl-2H-chromen,
4-Brommethyl-2-methyl-2H-chromen,
4-Brommethyl-2,2-dimethylchromen,
4-Brommethyl-2-isopropyl-2H-chromen,
4-Chlormethyl-2,2-diethylchromen,
4-Chlormethyl-2-methyl-2-propylchromen,
4-Brommethyl-2-hexyl-2H-chromen,
4-Brommethyl-2-cyclopentyl-2H-chromen,
4-Brommethyl-2-cyclohexyl-2H-chromen,
4-Brommethyl-2-spirocyclopentachromen,
4-Brommethyl-2-spirocyclohexachromen,
4-Brommethyl-6-methyl-2-spirocyclopentachromen,
4-Brommethyl-7-methyl-2-spirocyclopentachromen,
4-Brommethyl-6,8-dimethyl-2-spirocyclopentachromen,
4-Brommethyl-3-chlor-2-spirocyclopentachromen,

4-Brommethyl-6-methoxy-2-spirocyclopentachromen,
4-Brommethyl-7-methoxy-2-spirocyclopentachromen,
4-Brommethyl-7-isopropoxy-2-spirocyclopentachromen,
4-Brommethyl-7-phenoxy-2-spirocyclopentachromen,
4-Brommethyl-7-benzyloxy-2-spirocyclopentachromen,
4-Brommethyl-7-phenyl-2-spirocyclopentachromen,
4-Brommethyl-6-methyl-2,2-dimethylchromen,
4-Brommethyl-6-chlor-2,2-dimethylchromen,
4-Brommethyl-7-me thoxy-2,2-dimethylchromen,
4-Brommethyl-6-methyl-2-spirocyclohexachromen,
4-Brommethyl-7-methoxy-2-spirocyclohexachromen,
4-(p-Tosyloxymethyl)-2-spirocyclopentachroman
4-Mesyloxymethyl-2-spirocyclopentachroman.

Die erfindungsgemäßen Chroman- und Chromen-Derivate zeigen überraschenderweise eine antihypertensive Wirkung und können deshalb in freier Form oder in Form ihrer pharmazeutisch unbedenklichen Säureadditionssalze als Arzneimittel eingesetzt werden.

Die neuen Verbindungen haben ein breites und vielseitiges pharmakologisches Wirkungsspektrum und überraschend lange Wirkungsdauer.

Im einzelnen konnten im Tierexperiment folgende Hauptwirkungen nachgewiesen werden:

1. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten manifestieren. Die Verbindungen eignen sich daher besonders als Cerebraltherapeutika.

2. Die Verbindungen senken den Blutdruck von normotonen und hypertonen Tieren und können somit als antihypertensive Mittel verwendet werden.

Die erfindungsgemäßen Verbindungen eignen sich insbesondere zur Prophylaxe und Heilung von akuten und chronischen Herzkrankheiten, zur Therapie des Hochdrucks sowie zur Behandlung von cerebralen und peripheren Funktions- und Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesam-Öl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und dem individuellen Verhalten gegenüber dem Medikament bzw. der Art von dessen Formulierung und dem Zeipunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungspielraum vorgesehen. Sinngemäß gelten hierbei auch die obigen Ausführungen.

**Erfindungsgemäße Beispiele**

Herstellung nach Verfahrensvariante A

**Beispiel 1**

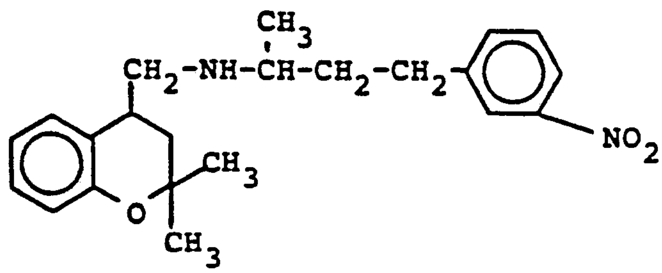

19,0 g (0,1 m) 2,2-Dimethyl-4-formylchroman werden in 100 ml Methanol unter Rühren und schwacher Kühlung bei 20°C mit 12,1 g (0,1 m) 2-Amino-4-(3-nitrophenyl)-butan versetzt und 1,5 Stunden gerührt. Dann fügt man 4,5 g Natriumboranat hinzu und läßt 1 Tag weiterrühren, engt ein und arbeitet mit Essigester/Wasser auf. Die Essigesterphase wird mit Wasser gewaschen, getrocknet und eingeengt. Destillation ergibt 4-{N-[4-(3-Nitrophenyl)-2-butyl]-aminomethyl}-2,2-Dimethylchroman. Kp: 225 - 235°C/0,04 mmHg.

**Beispiel 2** (Herstellung nach Verfahrensvariante B)

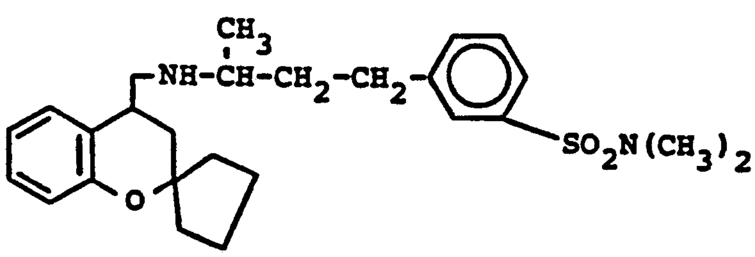

Eine Lösung von 6,5 g 2,2-Spirocyclopentan-4-aminomethylchroman, 9 g 4-(m-Dimethylaminosulfonyl)-phenylbutanon-(2) und 50 ml Toluol wird 2 Stunden am Wasserabscheider erhitzt und dann eingeengt. Der Rückstand wird in 150 ml Methanol verrührt und bei < 25°C mit 5 g Natriumboranat versetzt. Nach 2 Tagen engt man ein, verteilt den Rückstand zwischen Wasser und Toluol und engt die Toluolphase ein. Der Rückstand (14,5 g) wird in Ether gelöst und mit einer Lösung von 3 g Maleinsäure in 35 ml Tetrahydrofuran versetzt. Der entstehende Niederschlag wird nach 1 Tag abgesaugt und getrocknet, wobei 11,5 g des bei 113 - 120°C schmelzenden Maleinsäure-Salzes des 2,2-Spirocyclopentan-4-[N-(4-m-dimethylaminosulfonylphenyl)-2-butyl]-aminomethylchromans entstehen.

**Beispiel 3** (Herstellung nach Verfahrensvariante B)

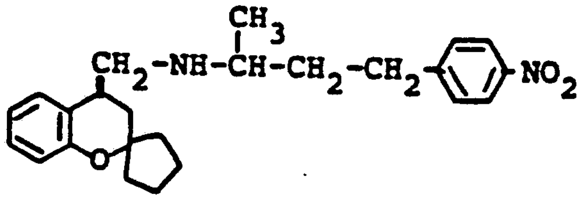

21,73 g (0,1 Mol) 2-Spirocyclopenta-4-aminomethylchroman werden mit 19,3 g (0,1 Mol) 4-(4-Nitrophenyl)-butanon-(2) in 150 ml Toluol bis zum Ende der Wasserabscheidung gekocht. Es wird eingedampft, in 100 ml abs. Methanol aufgenommen und portionsweise mit 3,78 g (0,1 Mol) Natriumborhydrid versetzt. Nach 2 h bei Raumtemperatur wird eingedampft, der Rückstand wird zwischen 50 ml $CH_2Cl_2$ und 50 ml Wasser verteilt, die organische Phase 1 x mit 2 n NaOH gewaschen, über $Na_2SO_4$ getrocknet und wieder eingedampft. Der Rückstand wird im Kugelrohr im Hochvakuum destilliert oder über Kieselgel mit $CH_2Cl_2$/Aceton 2 : 1 chromatographiert. Sdp.: 250° C/0,3 mm Hg Ausbeute: 24,63 g (63 g der Theorie).

**Beispiel 4**

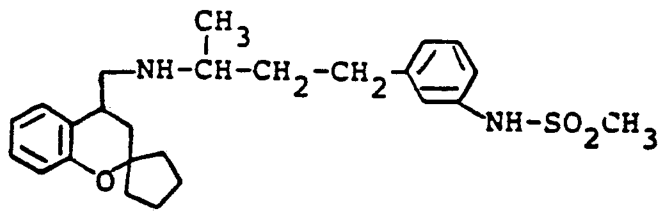

x HCl

21,73 g (0,1 Mol) 2-Spirocyclopenta-4-aminomethylchroman werden mit 24 g (0,1 Mol) 4-(3-Methylsulfonylaminophenyl)-butanon-(2) wie in Beispiel 3 umgesetzt. Der Rückstand wird über Kieselgel mit $CH_2Cl_2$/Aceton im Verhältnis 2 : 1 rasch chromatographiert, die Produkte enthaltenden Fraktionen werden eingedampft und der Rückstand zwischen $CH_2Cl_2$ und 10 % HCl verteilt. Nach Stehen über Nacht wird das zwischen den beiden Phasen ausgefallene Hydrochlorid abfiltriert, mit Ether nachgewaschen und getrocknet. Ausbeute 28,3 g (59 % der Theorie), Fp.: 218 - 231° C (Hydrochlorid).

**Beispiel 5** (Herstellung nach Verfahrensvariante C)

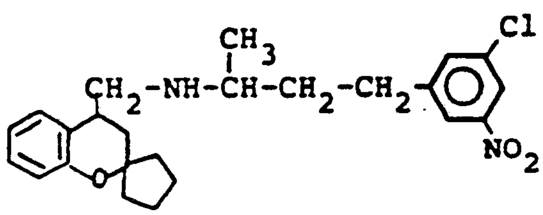

3 g (15,2 mmol) 2-Spirocyclopenta-4-p-tosyloxymethylchroman werden zusammen mit 2,94 g (15,2 mmol) 4-(3-Chlor-5-nitrophenyl)-2-aminobutan und 2,1 g (15,2 mmol) $K_2CO_3$ in 30 ml Acetonitril 10 h refluxiert. Es wird eingedampft, in 60 ml $CH_2Cl_2$ aufgenommen und 1 x mit 60 ml 2n HCl, 1 x mit 60 ml 2 n NaOH und 1 x mit 60 ml $H_2O$ gewaschen. Nach Trocknen über $Na_2SO_4$ wird eingedampft und bei 200 - 210° C/0,1 mm Hg destilliert.
Ausbeute: 3,05 g (51 %)

Die analoge Umsetzung von 10,86 g 2-Spirocyclopenta-4-aminomethylchroman mit 13,61 g 4-(3,4-Methylendioxyphenyl)-butanol-2-mesylat gibt 5,91 g (30,1 g der Theorie) Produkt.

**Beispiel 6**

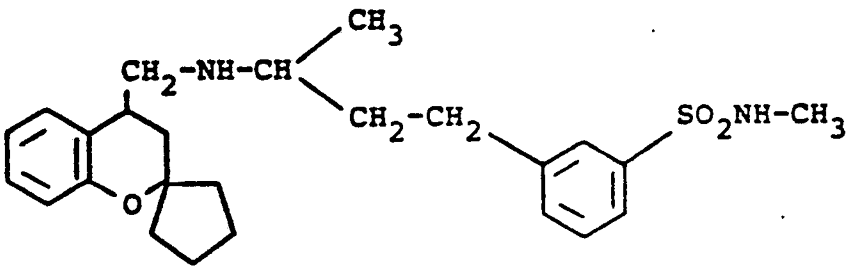

Öl;

**Beispiel 7**

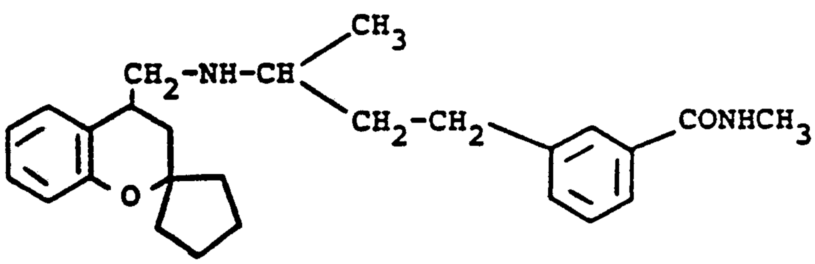

Öl; Sdp.: 270° C/0,01 torr

**Beispiel 8**

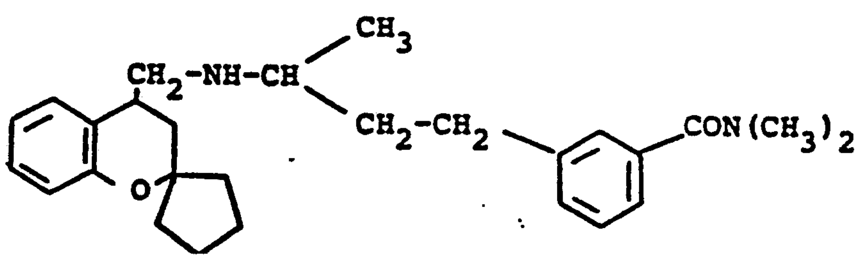

Öl; Sdp.: 260 - 270° C/0,01 torr

**Beispiel 9**

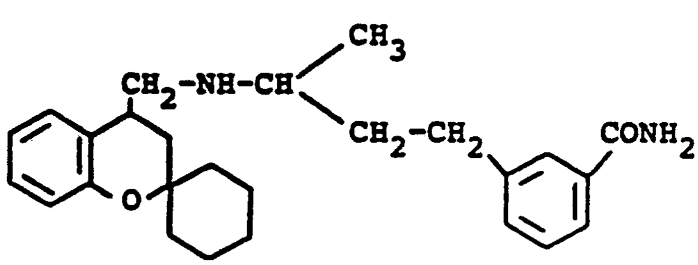

Öl; Sdp.: 270 - 280° C/0,01

**Beispiel 10**

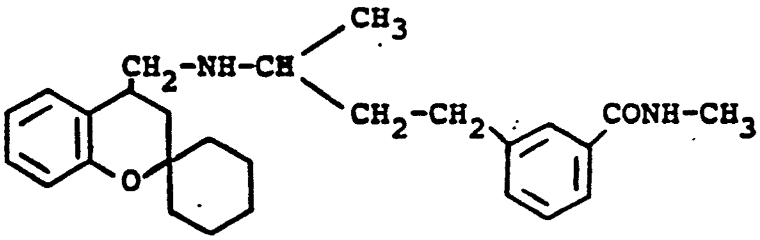

Öl; Sdp.: 260 - 270° C/0,02

**Beispiel 11**

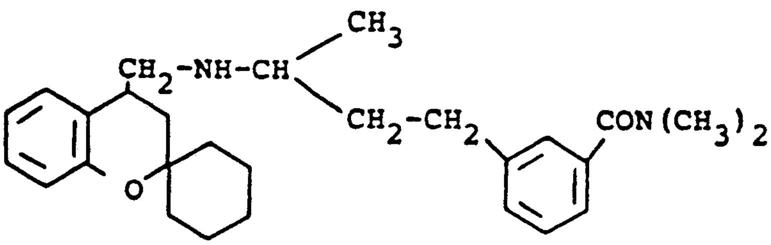

Öl; Sdp.: 260° C/0,01 mmHg

**Beispiel 12**

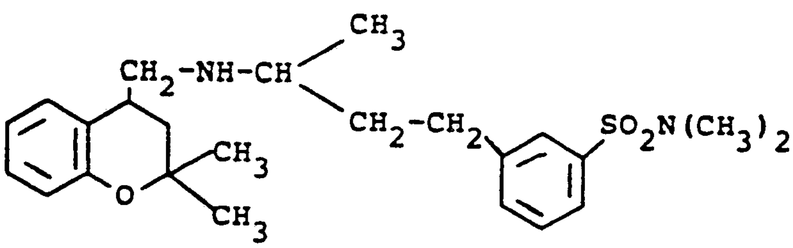

Öl;

**Beispiel 13**

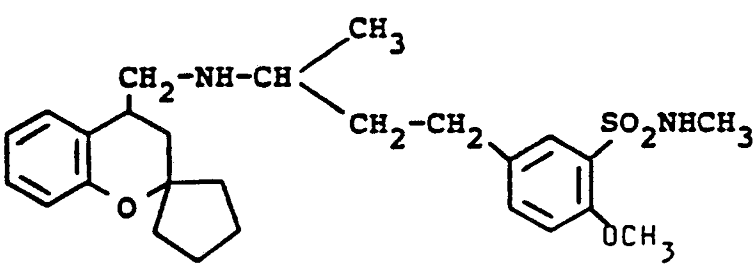

Öl;

**Beispiel 14**

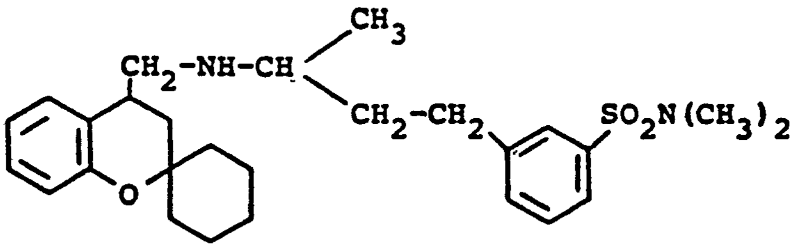

Öl;

**Beispiel 15**

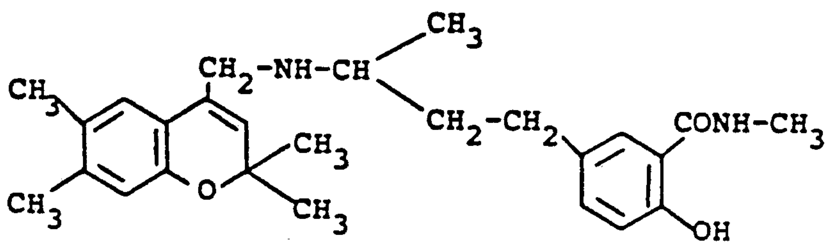

Öl;

**Beispiel 16**

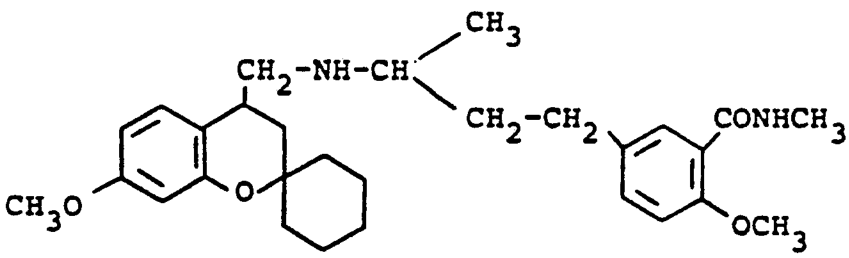

Öl;

**Beispiel 17**

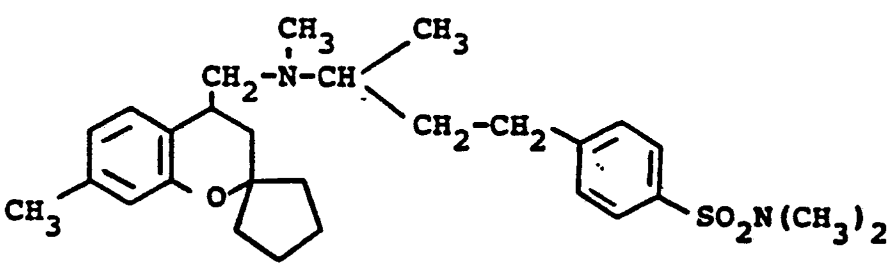

Öl;

**Beispiel 18**

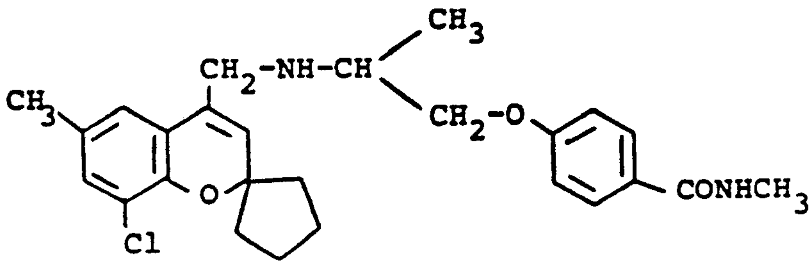

Öl;

**Beispiel 19**

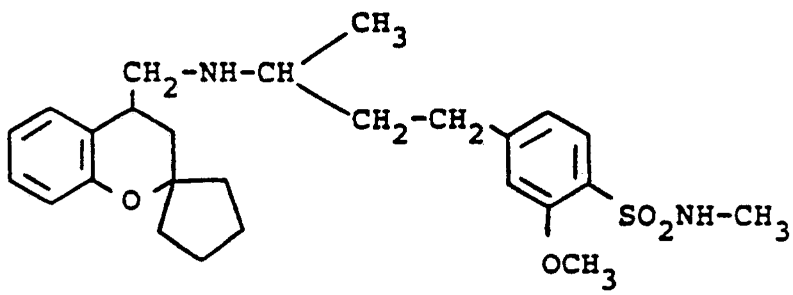

Öl;

**Beispiel 20**

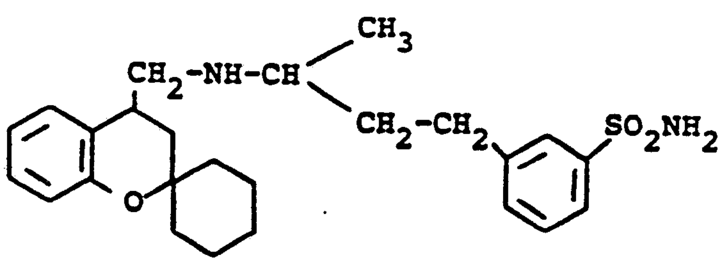

Öl;

**Beispiel 21**

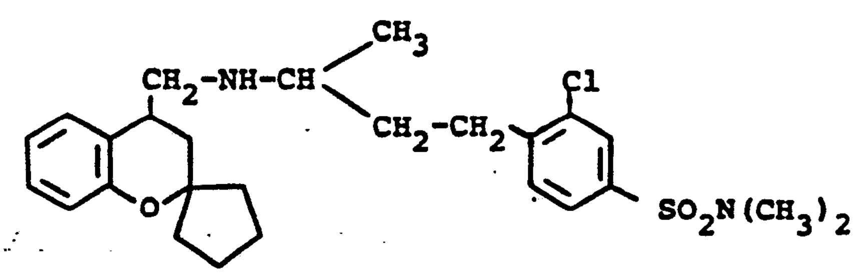

Öl;

**Beispiel 22**

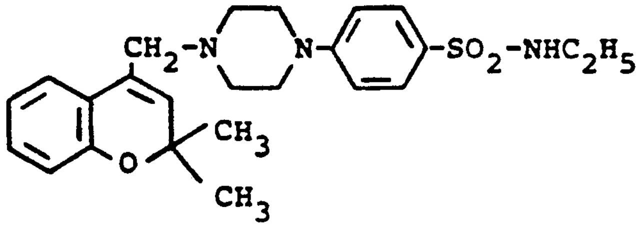

HCl-Salz; Fp.: 100 - 120° C

**Beispiel 23**

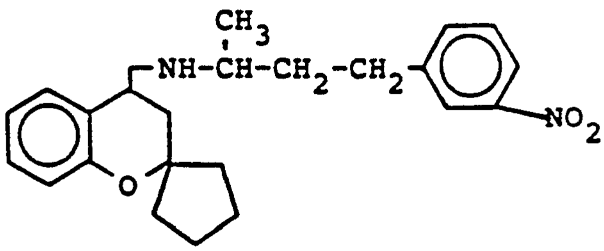

Kp.: 225 - 235° C/0,04 mmHg

**Beispiel 24**

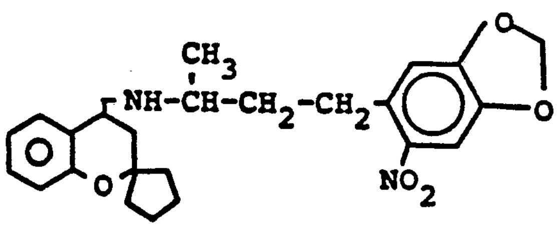

Fp.: 172 - 175° C (Fumarat)

**Beispiel 25**

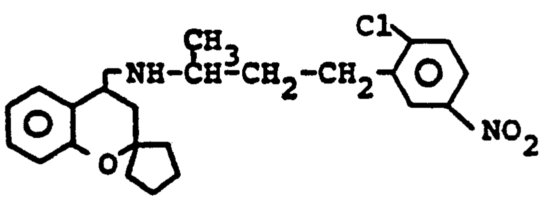

Kp.: 220° C/0,01 mmHg

**Beispiel 26**

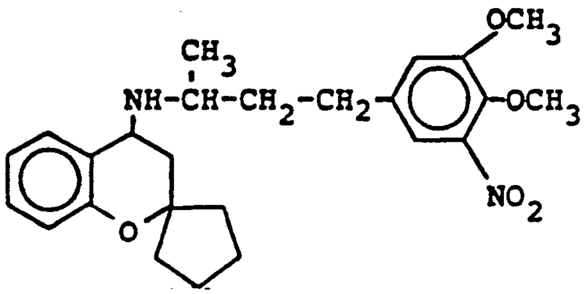

Kp.: 240° C/0,01 mmHg

**Beispiel 27**

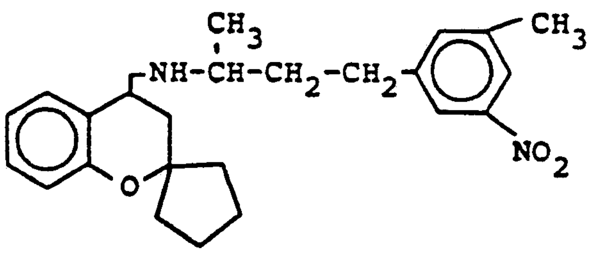

Kp.: 245 - 250° C/0,5 mmHg

**Beispiel 28**

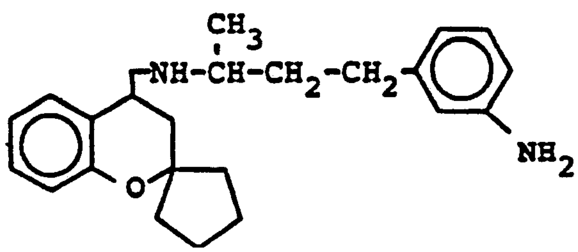

Fp.: 119 - 123° C (Maleat)

**Beispiel 29**

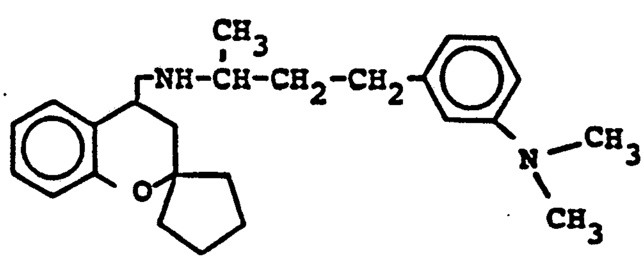

Öl;

**Beispiel 30**

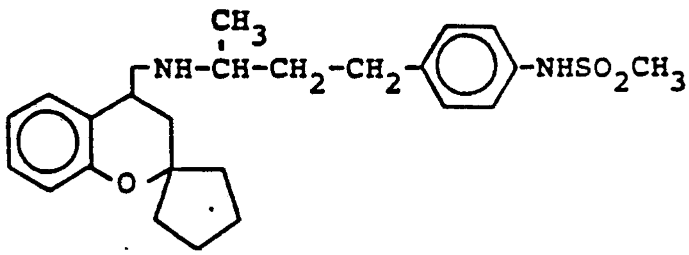

Öl; Kp: 280° C/0,1 mmHg

**Beispiel 31**

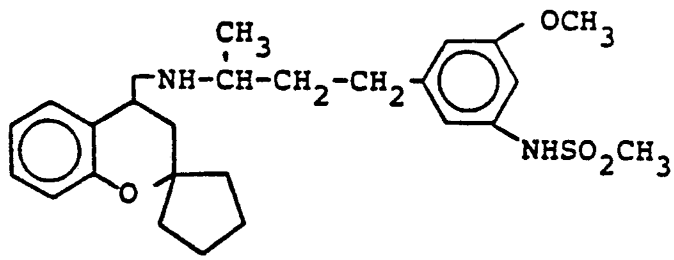

Öl;

**Beispiel 32**

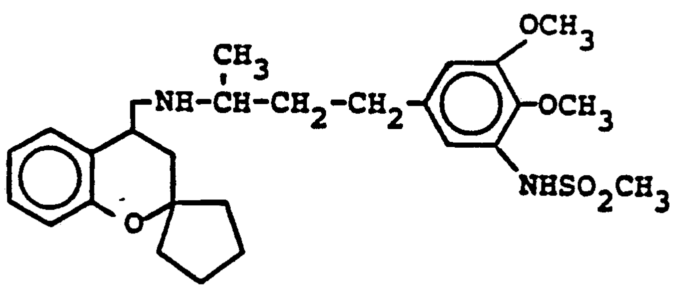

Öl; Fp: 120 - 122° C (Maleat)

**Beispiel 33**

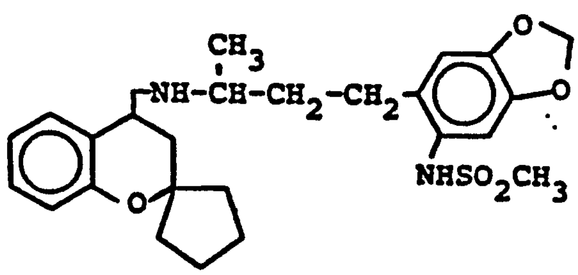

Fp.: 149 - 151° C (Maleat)

**Beispiel 34**

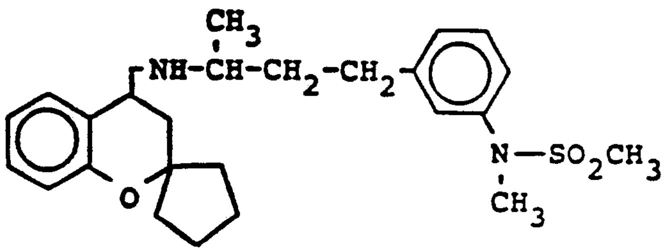

Fp.: 169 - 171° C (Zers.) Fumarat

**Beispiel 35**

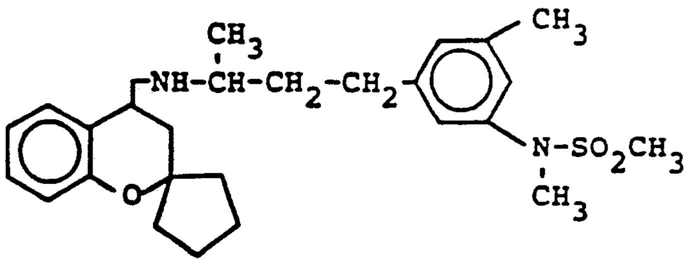

Fp.: 164 - 166° C (Maleat)

**Beispiel 36**

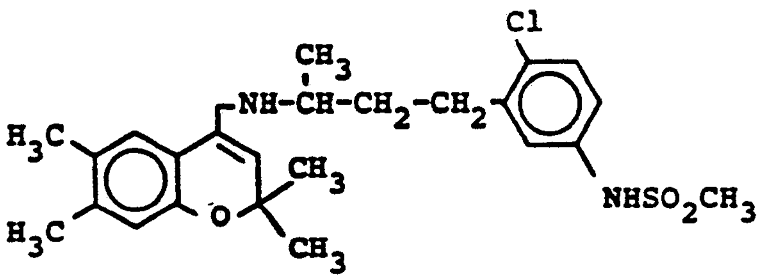

Öl;

**Beispiel 37**

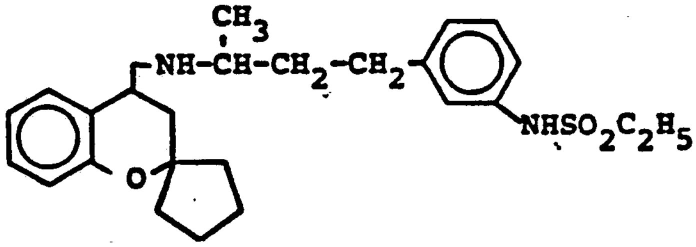

Fp.: 120 - 124° C (Zers. Fumarat)

**Beispiel 38**

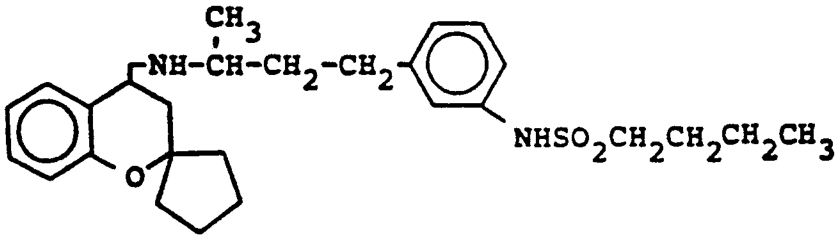

Fp.: 119 - 120° C (Zerzs. Fumarat)

**Beispiel 39**

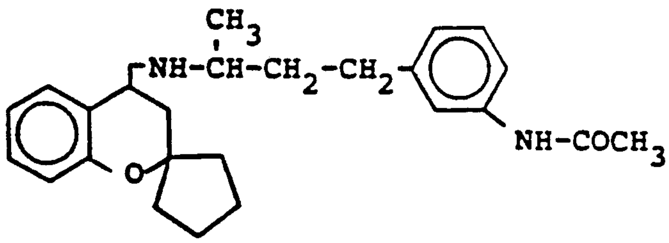

Öl; Kp: 225° C/0,1 mmHg

**Beispiel 40**

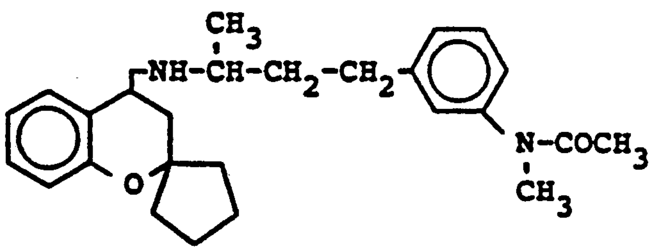

Öl;

**Beispiel 41**

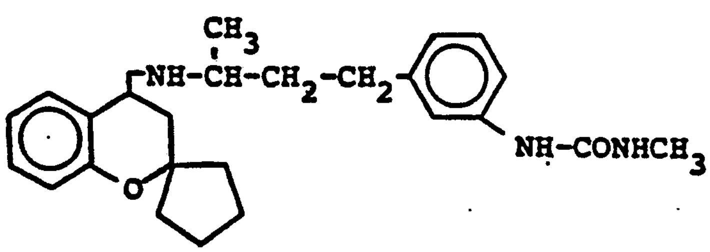

Öl;

**Beispiel 42**

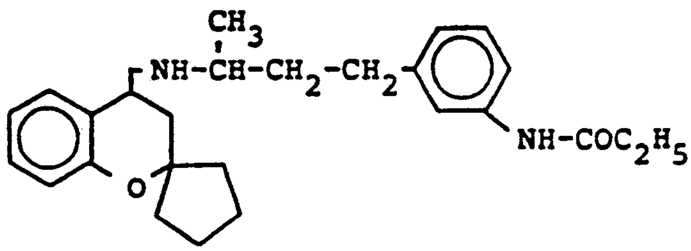

Fp.: 132° C (Zers. Fumarat)

**Beispiel 43**

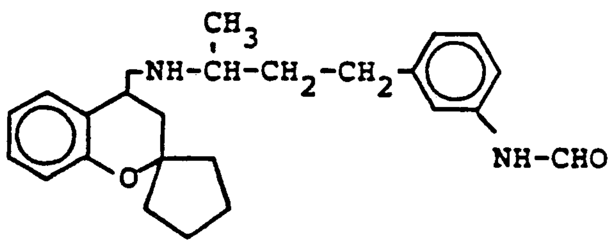

Fp.: 119° C (Zers. Fumarat)

**Beispiel 44**

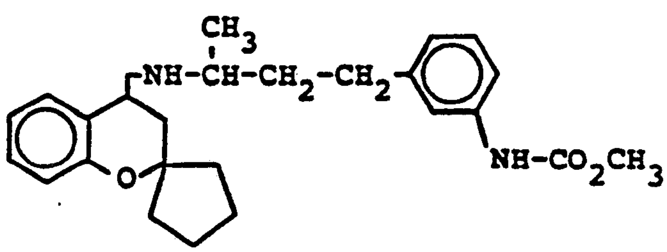

Öl;

**Beispiel 45**

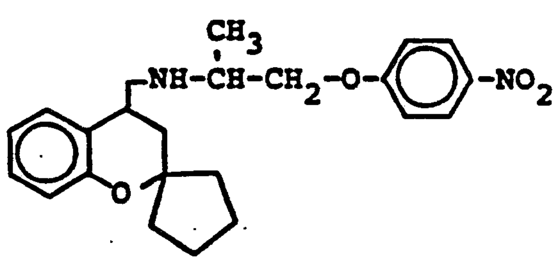

Fp.: 175 - 179° C (Maleat)

**Beispiel 46**

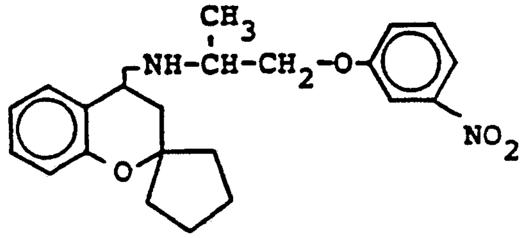

Fp.: 145 - 150° C (Maleat)

**Beispiel 47**

5

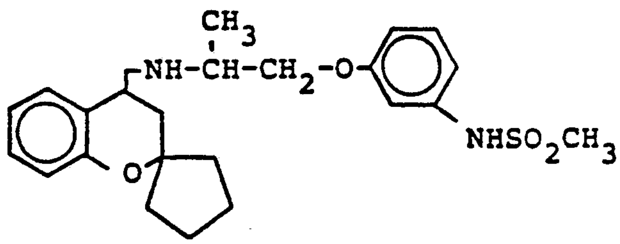

Öl;

**Beispiel 48**

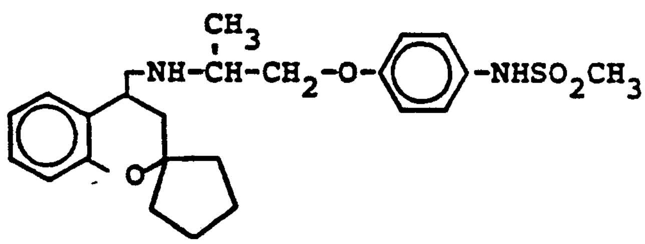

Fp.: 153 - 155° C (Maleat)

**Beispiel 49**

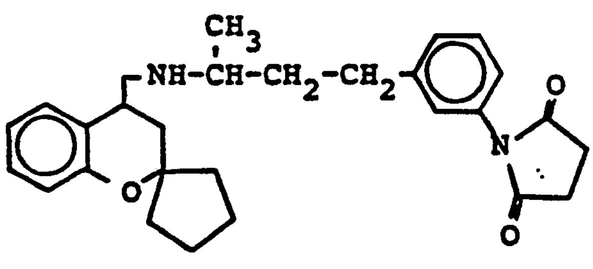

Öl; Kp: 280 - 300° C/0,1 mmHg

**Beispiel 50**

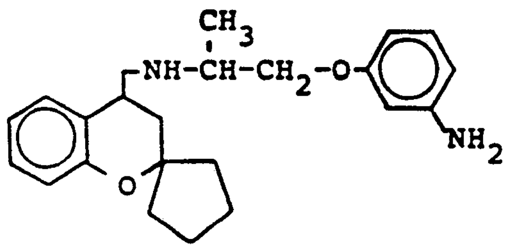

Öl;

**Beispiel 51**

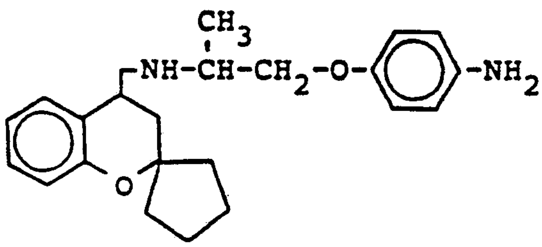

Öl;

**Beispiel 52**

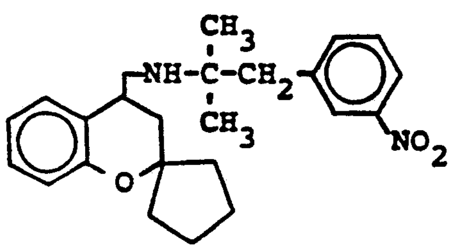

Öl;

**Beispiel 53**

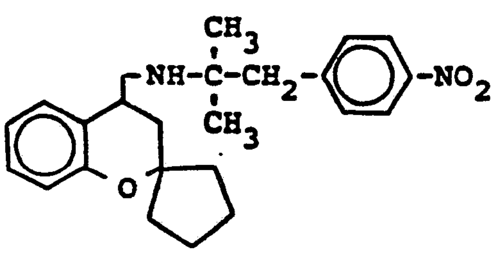

Öl;

**Beispiel 54**

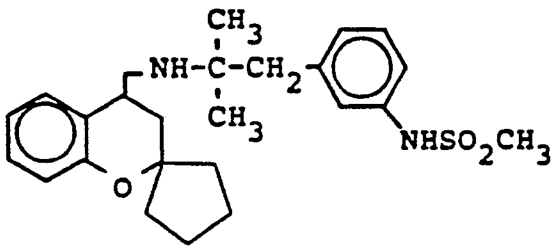

Öl;

**Beispiel 55**

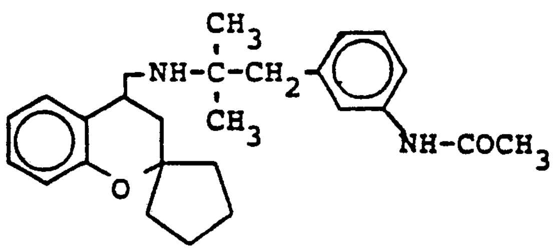

Öl;

**Beispiel 56**

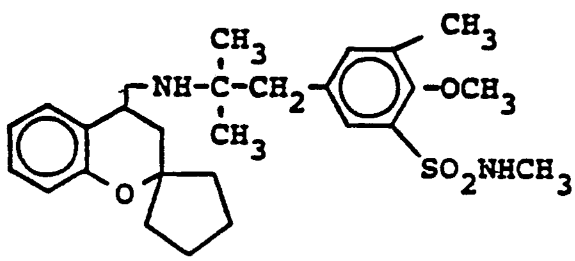

Öl;

**Beispiel 57**

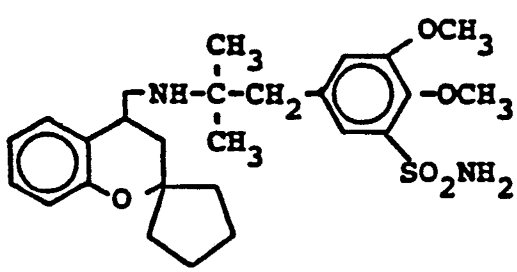

Öl;

**Beispiel 58**

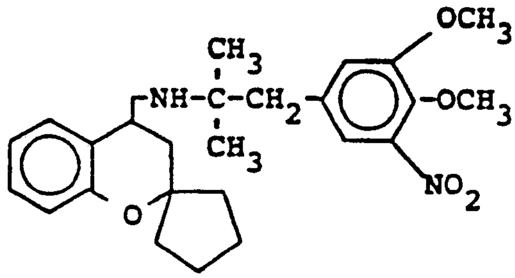

Öl;

**Beispiel 59**

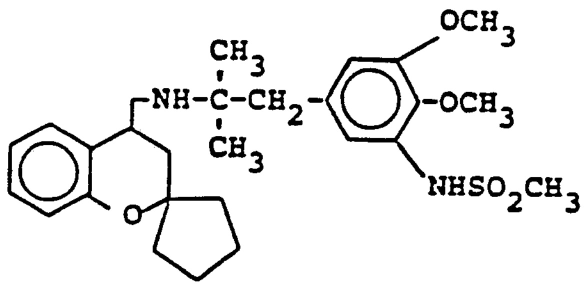

Öl;

**Beispiel 60**

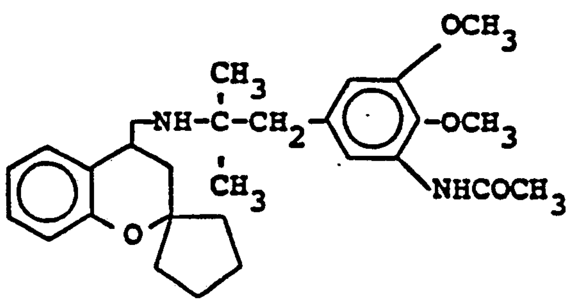

Öl;

**Beispiel 61**

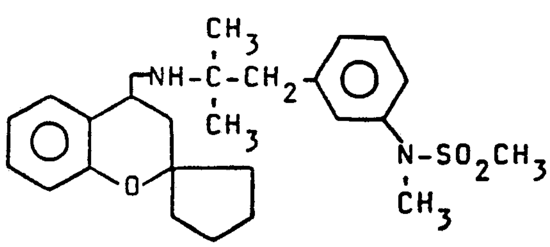

Öl;

**Patentansprüche**

1. Substituierte Benzopyrane der allgemeinen Formel (I)

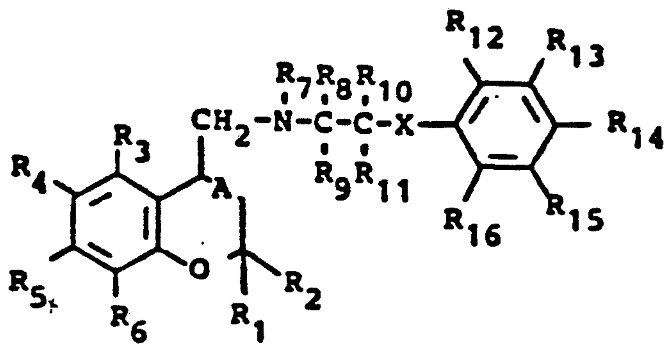

in der

-A- eine Einfachbindung oder eine Doppelbindung darstellt,

$R_1$ und $R_2$ gleich oder verschieden, für Wasserstoff, $C_1$-$C_6$-Alkyl, $C_5$-$C_7$-Cycloalkyl, Phenyl, gegebenenfalls ein- oder zweifach substituiert durch $C_1$-$C_4$-Alkyl, Halogen und/oder $C_1$-$C_4$-Alkoxy, für $C_7$-$C_9$-Aralkyl, dessen Arylrest gegebenenfalls 1- oder 2-fach substituiert ist durch $C_1$-$C_4$-Alkyl, Halogen und/oder $C_1$-$C_4$-Alkoxy stehen, oder in welcher $R_1$ und $R_2$ zusammen mit dem eingeschlossenen C-Atom des Chromanringes einen 4- bis 7-gliedrigen carbocyclischen Ring bilden;

$R_3$ bis $R_6$, gleich oder verschieden, für Wasserstoff, Hydroxy, Halogen, $C_1$-$C_6$-Alkyl, $C_5$-$C_7$-Cycloalkyl, Phenyl, gegebenenfalls 1- bis 2-fach substituiert durch $C_1$-$C_4$-Alkyl, Halogen und/oder $C_1$-$C_4$-Alkoxy oder für $C_7$-$C_9$-Aralkyl, dessen Arylrest gegebenenfalls 1- bis 2-fach substituiert ist durch $C_1$-$C_4$-Alkyl, Halogen und/oder $C_1$-$C_4$-Alkoxy, stehen;

$R_7$, $R_8$, $R_9$, $R_{10}$, $R_{11}$ gleich oder verschieden sind und für Wasserstoff oder $C_1$-$C_6$-Alkyl stehen;

X für eine Einfachbindung, für Methylen, gegebenenfalls ein- oder zweifach substituiert durch $C_1$-$C_4$-Alkyl, für Sauerstoff oder für $-NR_{17}$ steht, wobei $R_{17}$ Wasserstoff oder $C_1$-$C_4$-Alkyl darstellt oder $R_{17}$ zusammen mit $R_7$ für $C_2$-Alkylen steht;

mindestens einer und höchstens zwei der Reste

$R_{12}$, $R_{13}$, $R_{14}$, $R_{15}$ und $R_{16}$, gleich und verschieden, für Nitro, Cyano oder eine der Gruppen

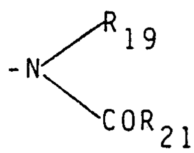 , 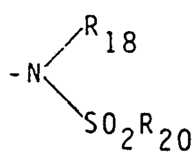 , 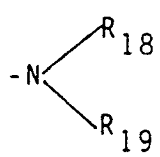 ,

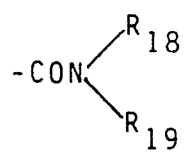 oder 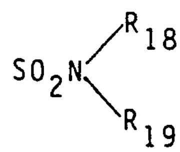 stehen, worin

stehen, worin

$R_{18}$, $R_{19}$ gleich oder verschieden sind und jeweils für Wasserstoff oder Alkyl mit 1 - 4 C-Atomen stehen, welches gegebenenfalls 1- bis 3-fach substituiert ist durch Halogen,

$R_{20}$ für Alkyl mit 1-6 C-Atomen steht, welches gegebenenfalls 1- bis 3-fach substituiert ist durch Halogen, und

$R_{21}$ für Wasserstoff, Alkyl, Alkoxy oder Alkylamino mit jeweils 1-4 C-Atomen pro Alkyl und Alkoxygruppe steht, wobei der Alkylrest gegebenenfalls 1- bis 3-fach substituiert ist durch Halogen,

oder worin

$R_{18}$ gemeinsam mit $R_{19}$ oder gemeinsam mit $R_{20}$ oder gemeinsam mit $R_{21}$ einen 5- bis 6-gliedrigen heterocyclischen Ring bilden kann, der eine Alkylbrücke mit 2, 3 oder 4 Kohlenstoffatomen enthält und gegebenenfalls noch eine zusätzliche Carbonylgruppe enthält,

während die übrigen Reste $R_{12}$ bis $R_{16}$ gleich oder verschieden sind und jeweils für Wasserstoff, Hydroxy, Chlor, Fluor, Alkyl mit 1 bis 4 C-Atomen, Alkoxy mit 1 bis 3 C-Atomen, Aralkyloxy mit bis zu 8 C-Atomen, oder Trifluormethyl stehen,

und überdies

$R_{12}$ und $R_{13}$ oder $R_{13}$ und $R_{14}$ gemeinsam für eine Alkylendioxygruppe mit 1 oder 2 C-Atomen oder die Gruppe -CH=CH-CH=CH- stehen können;

sowie ihre pharmazeutisch unbedenklichen Additionssalze.

2. Verbindungen der allgemeinen Formel (I) gemäß Anspruch 1, in welcher

$R_1$ und $R_2$ gleich oder verschieden sind und für Wasserstoff, $C_1$-$C_4$-Alkyl stehen oder zusammen mit dem von ihnen eingeschlossenen Kohlenstoffatom einen carbocyclischen $C_5$ oder $C_6$ Ring bilden,

$R_3$ bis $R_6$ gleich oder verschieden sind und Wasserstoff, Hydroxy, $C_1$-$C_4$-Alkyl, $C_1$-$C_4$-Alkoxy oder Chlor bedeuten,

$R_7$ bis $R_{11}$ gleich oder verschieden sind und Wasserstoff oder $C_1$-$C_4$-Alkyl darstellen,

X für eine Einfachbindung, für Sauerstoff, für Methylen oder für -$NR_{17}$ steht, wobei $R_{17}$ Wasserstoff oder $C_1$-$C_3$-Alkyl bedeutet, oder wobei $R_{17}$ zusammen mit $R_7$ einen Ethylenringschluß bildet, und

$R_{12}$ bis $R_{16}$ gleich oder verschieden sind und Wasserstoff, Chlor, Cyclohexyl, $C_1$-$C_4$-Alkyl, $C_1$-$C_3$-Alkoxy, Aralkoxy mit 1-8 C-Atomen, Trifluormethyl bedeuten, wobei $R_{12}$ und $R_{13}$ oder $R_{13}$ und $R_{14}$ gegebenenfalls zusammen jeweils eine Methylendioxygruppe oder eine -CH = CH-CH = CH-Gruppe bilden,

und wobei mindestens eine und höchstens zwei Substituenten aus der Gruppe $R_{12}$ - $R_{16}$ gleich und verschieden sind und jeweils für Nitro, Cyano, die Gruppe

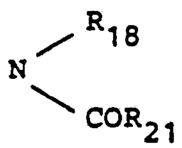 , 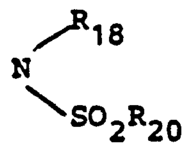 , 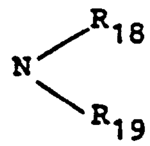 , 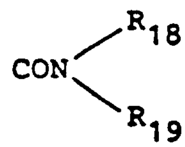 ,

oder 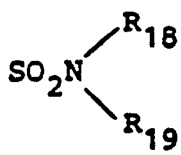 stehen, wobei $R_{18}$ und $R_{19}$

stehen, wobei

$R_{18}$ und $R_{19}$ gleich oder verschieden sind und jeweils für Wasserstoff oder gegebenenfalls durch Halogen substituiertes Alkyl mit 1-4 C-Atomen stehen und wobei $R_{20}$ für gegebenenfalls durch Fluor oder Chlor substituiertes Alkyl mit 1-4 C-Atomen steht und wobei $R_{21}$ für Wasserstoff, gegebenenfalls durch Fluor oder Chlor substituiertes Alkyl mit 1-4 C-Atomen, Alkoxy oder Alkylamino mit jeweils 1-2 C-Atomen steht.

3. Verbindungen der allgemeinen Formel (I) gemäß Anspruch 1 zur Verwendung bei der Bekämpfung von Erkrankungen.

4. Verfahren zur Herstellung von Verbindungen der allgemeinen Formel (I)

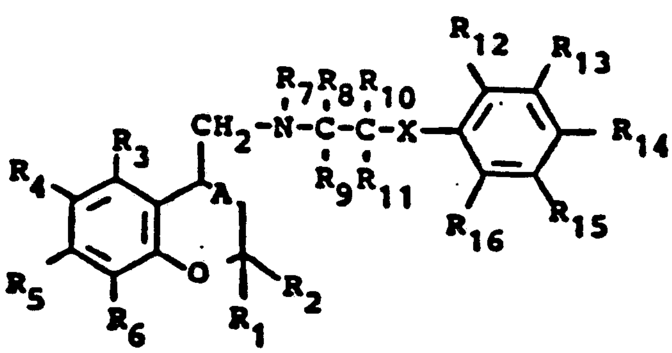

-A- eine Einfachbindung oder eine Doppelbindung darstellt,

$R_1$ und $R_2$ gleich oder verschieden, für Wasserstoff, $C_1$-$C_6$-Alkyl, $C_5$-$C_7$-Cycloalkyl, Phenyl, gegebenenfalls ein- oder zweifach substituiert durch $C_1$-$C_4$-Alkyl, Halogen und/oder $C_1$-$C_4$-Alkoxy, für $C_7$-$C_9$-Aralkyl, dessen Arylrest gegebenenfalls 1- oder 2-fach substituiert ist durch $C_1$-$C_4$-Alkyl, Halogen und/oder $C_1$-$C_4$-Alkoxy stenen, oder in welcher $R_1$ und $R_2$ zusammen mit dem eingeschlossenen C-Atom des Chromanringes einen 4- bis 7-gliedrigen carbocyclischen Ring bilden;

$R_3$ bis $R_6$, gleich oder verschieden, für Wasserstoff, Hydroxy, Halogen, $C_1$-$C_6$-Alkyl, $C_5$-$C_7$-Cycloalkyl, Phenyl, gegebenenfalls 1- bis 2-fach substituiert durch $C_1$-$C_4$-Alkyl, Halogen und/oder $C_1$-$C_4$-Alkoxy oder für $C_7$-$C_9$-Aralkyl, dessen Arylrest gegebenenfalls 1- bis 2-fach substituiert ist durch $C_1$-$C_4$-Alkyl, Halogen und/oder $C_1$-$C_4$-Alkoxy, stehen;

$R_7$, $R_8$, $R_9$, $R_{10}$, $R_{11}$ gleich oder verschieden sind und für Wasserstoff oder $C_1$-$C_6$-Alkyl stehen;

X für eine Einfachbindung, für Methylen, gegebenenfalls ein- oder zweifach substituiert durch $C_1$-$C_4$-Alkyl, für Sauerstoff oder für -$NR_{17}$ steht, wobei $R_{17}$ Wasserstoff oder $C_1$-$C_4$-Alkyl darstellt oder $R_{17}$ zusammen mit $R_7$ für $C_2$-Alkylen steht;

mindestens einer und höchstens zwei der Reste
$R_{12}$, $R_{13}$, $R_{14}$, $R_{15}$ und $R_{16}$, gleich und verschieden, für Nitro, Cyano oder eine der Gruppen

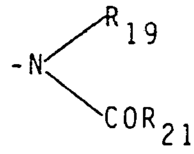 , 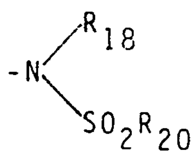 , 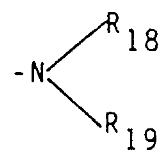 ,

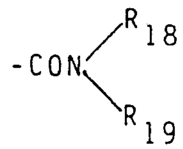 oder 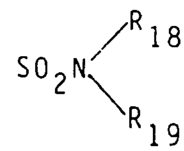

stehen, worin
$R_{18}$ und $R_{19}$ gleich oder verschieden sind und jeweils für Wasserstoff oder Alkyl mit 1 - 4 C-Atomen stehen, welches gegebenenfalls 1- bis 3-fach substituiert ist durch Halogen,
$R_{20}$ für Alkyl mit 1-6 C-Atomen steht, welches gegebenenfalls 1- bis 3-fach substituiert ist durch Halogen, und
$R_{21}$ für Wasserstoff, Alkyl, Alkoxy oder Alkylamino mit jeweils 1-4 C-Atomen pro Alkyl und Alkoxygruppe steht, wobei der Alkylrest gegebenenfalls 1- bis 3-fach substituiert ist durch Halogen,
oder worin
$R_{18}$ gemeinsam mit $R_{19}$ oder gemeinsam mit $R_{20}$ oder gemeinsam mit $R_{21}$ einen 5- bis 6-gliedrigen heterocyclischen Ring bilden kann, der eine Alkylbrücke mit 2, 3 oder 4 Kohlenstoffatomen enthält und gegebenenfalls noch eine zusätzliche Carbonylgruppe enthält,
während die übrigen Reste $R_{12}$ bis $R_{16}$ gleich oder verschieden sind und jeweils für Wasserstoff, Hydroxy, Chlor, Fluor, Alkyl mit 1 bis 4 C-Atomen, Alkoxy mit 1 bis 3 C-Atomen, Aralkyloxy mit bis zu 8 C-Atomen, oder Trifluormethyl stehen,
und überdies
$R_{12}$ und $R_{13}$ oder $R_{13}$ und $R_{14}$ gemeinsam für eine Alkylendioxygruppe mit 1 oder 2 C-Atomen oder die Gruppe -CH=CH-CH=CH- stehen können;
sowie ihrer pharmazeutisch unbedenklichen Additionssalze, dadurch gekennzeichnet, daß man

A) Chroman-4-carbaldehyde der Formel (II)

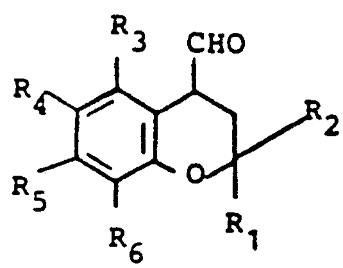

in welcher
$R_1$, $R_2$, $R_3$, $R_4$, $R_5$ und $R_6$ die oben angegebene Bedeutung haben,
mit Aminen der Formel (III)

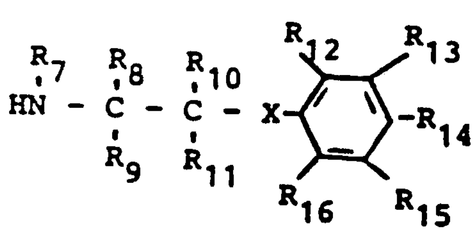

in welcher
$R_7$, $R_8$, $R_9$, $R_{10}$, $R_{11}$, $R_{12}$, $R_{13}$, $R_{14}$, $R_{15}$, $R_{16}$, $R_{17}$ und X die oben angegebene Bedeutung haben,
in Gegenwart von reduzierenden Agentien umsetzt,
oder

B) Amine der Formel (IV)

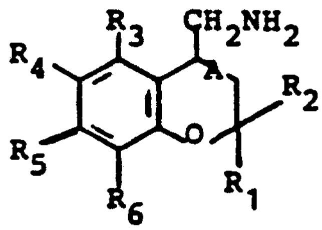

in welcher
A, $R_1$, $R_2$, $R_3$, $R_4$, $R_5$ und $R_6$ die oben angegebene Bedeutung haben, mit Carbonylverbindungen der Formel (V)

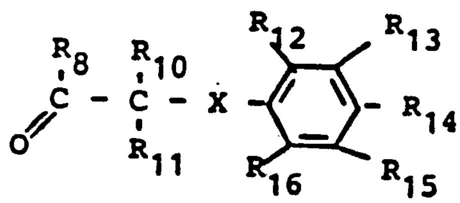

in welcher
$R_8$ bis $R_{16}$ die oben angegebene Bedeutung haben, mit der Maßgabe, daß X nicht für $NR_{17}$ steht, in Gegenwart von reduzierenden Agentien umsetzt,
oder

C) Verbindungen der Formel (VI)

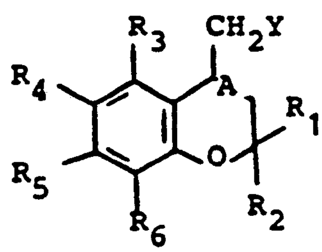

in welcher
A, $R_1$, $R_2$, $R_3$, $R_4$, $R_5$ und $R_6$ die oben angegebene Bedeutung haben, und
Y für nucleophil austauschbare Gruppen, wie Alkyl- oder Aryl substituierte Sulfonyloxygruppen, Brom oder Chlor steht,
mit den Aminen der Formel (III) in Gegenwart eines säurebindenden Mittels umsetzt.

5. Verfahren gemäß Anspruch 4, dadurch gekennzeichnet, daß man bei den Verfahrensvarianten A) und B) als reduzierende Agentien komplexe Metallhydride oder katalytisch angeregten Wasserstoff einsetzt und bei der Verfahrensvariante C) als säurebindende Mittel Metallhydroxide, Carbonate oder Stickstoffbasen einsetzt.

6. Verfahren gemäß Anspruch 4, dadurch gekennzeichnet, daß man die Umsetzungen bei Temperaturen zwischen - 50 und + 150° C durchführt.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 unter Verwendung von inerten Hilfs- und Trägerstoffen in eine geeignete Applikationsform überführt.

9. Verwendung von Verbindungen der allgemeinen Formel (I) gemäß Anspruch 1 zur Herstellung von Arzneimitteln zur Bekämpfung von Kreislauferkrankungen.

**Claims**

1. Substituted benzopyrans of the general formula (I)

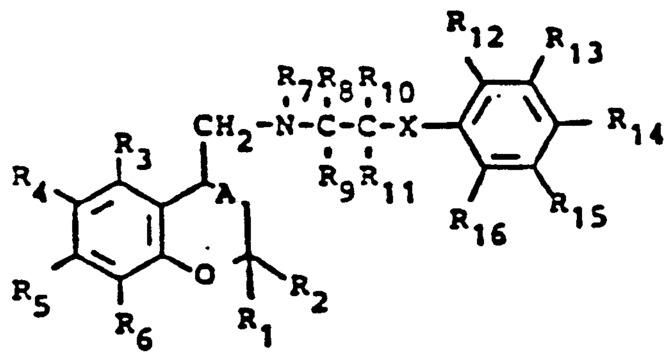

in which

-A- represents a single bond or a double bond, $R_1$ and $R_2$ are identical or different and represent hydrogen, $C_1$-$C_6$-alkyl, $C_5$-$C_7$-cycloalkyl, phenyl, optionally monosubstituted or disubstituted by $C_1$-$C_4$-alkyl, halogen and/or $C_1$-$C_4$-alkoxy, or $C_7$-$C_9$-aralkyl, the aryl radical of which is optionally monosubstituted or disubstituted by $C_1$-$C_4$-alkyl, halogen and/or $C_1$-$C_4$-alkoxy, or in which $R_1$ and $R_2$, together with the included C atom of the chroman ring, form a 4-membered to 7-membered carbocyclic ring;

$R_3$ to $R_6$ are identical or different and represent hydrogen, hydroxyl, halogen, $C_1$-$C_6$-alkyl, $C_5$-$C_7$-cycloalkyl, phenyl, optionally monosubstituted or disubstituted by $C_1$-$C_4$-alkyl, halogen and/or $C_1$-$C_4$-alkoxy, or $C_7$-$C_9$-aralkyl, the aryl radical of which is optionally monosubstituted or disubstituted by $C_1$-$C_4$-alkyl, halogen and/or $C_1$-$C_4$-alkoxy;

$R_7$, $R_8$, $R_9$, $R_{10}$ and $R_{11}$ are identical or different and represent hydrogen or $C_1$-$C_6$-alkyl,

X represents a single bond or methylene which is optionally monosubstituted or disubstituted by $C_1$-$C_4$-alkyl, or represents oxygen or $-NR_{17}$,

wherein

$R_{17}$ represents hydrogen or $C_1$-$C_4$-alkyl, or $R_{17}$ together with $R_7$ represents $C_2$-alkylene, wherein at least one and at most two of the radicals $R_{12}$, $R_{13}$, $R_{14}$, $R_{15}$ and $R_{16}$ are identical or different and represent nitro, cyano or one of the groups

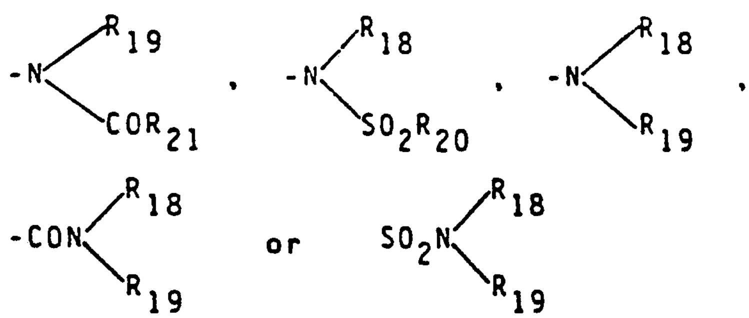

wherein

$R_{18}$ and $R_{19}$ are identical or different and each represent hydrogen or alkyl having 1 - 4 C atoms which is optionally monosubstituted to trisubstituted by halogen,

$R_{20}$ represents alkyl having 1-6 C atoms which is optionally monosubstituted to trisubstituted by halogen, and

$R_{21}$ represents hydrogen, alkyl, alkoxy or alkylamino, each having 1-4 C atoms per alkyl and alkoxy group, the alkyl radical being optionally monosubstituted to trisubstituted by halogen, or

wherein

$R_{18}$ together with $R_{19}$ or together with $R_{20}$ or together with $R_{21}$ can form a 5-membered to 6-membered heterocyclic ring which contains an alkyl bridge having 2, 3 or 4 carbon atoms and which optionally also contains an additional carbonyl group, whereas the remaining radicals $R_{12}$ to $R_{16}$ are identical or different and each represent hydrogen, hydroxyl, chlorine, fluorine, alkyl having 1 to 4 C atoms, alkoxy having 1 to 3 C atoms, aralkyloxy having up to 8 C atoms or trifluoromethyl,

and moreover

$R_{12}$ and $R_{13}$ or $R_{13}$ and $R_{14}$ together can represent an alkylenedioxy group having 1 or 2 C atoms or the group $-CH=CH-CH=CH-$,

and their pharmaceutically acceptable addition salts.

2. Compounds of the general formula (I) according to Claim 1,

in which

$R_1$ and $R_2$ are identical or different and represent hydrogen or $C_1$-$C_4$-alkyl, or $R_1$ and $R_2$, together with the carbon atom between them, form a carbocyclic $C_5$ or $C_6$ ring,

$R_3$ to $R_6$ are identical or different and denote hydrogen, hydroxyl, $C_1$-$C_4$-alkyl, $C_1$-$C_4$-alkoxy or chlorine,

$R_7$ to $R_{11}$ are identical or different and represent hydrogen or $C_1$-$C_4$-alkyl,

X represents a single bond, oxygen, methylene or $-NR_{17}$,

wherein

$R_{17}$ denotes hydrogen or $C_1$-$C_3$-alkyl, or

wherein

$R_{17}$ together with $R_7$ forms an ethylene ring closure, and

$R_{12}$ to $R_{16}$ are identical or different and denote hydrogen, chlorine, cyclohexyl, $C_1$-$C_4$-alkyl, $C_1$-$C_3$-alkoxy, aralkoxy having 1-8 C atoms or trifluoromethyl, and $R_{12}$ and $R_{13}$ or $R_{13}$ and $R_{14}$ together optionally each form a methylenedioxy group or a $-CH=CH-CH=CH-$ group,

and wherein

at least one and at most two substituents from the group comprising $R_{12}$ - $R_{16}$ are identical or different and each represent nitro, cyano or the

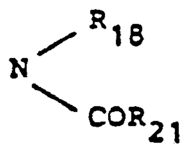 , 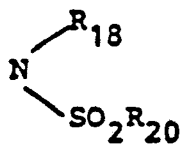 , 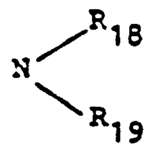 , 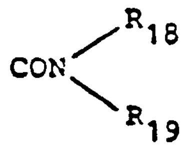 ,

or

or 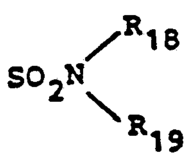

group

wherein

$R_{18}$ and $R_{19}$ are identical or different and each represent hydrogen or alkyl having 1-4 C atoms which is optionally substituted by halogen,

and wherein

$R_{20}$ represents alkyl having 1-4 C atoms which is optionally substituted by fluorine or chlorine,

and wherein

$R_{21}$ represents hydrogen or alkyl having 1-4 C atoms which is optionally substituted by fluorine or chlorine, or represents alkoxy or alkylamino, each having 1-2 C atoms.

3. Compounds of the general formula (I) according to Claim 1 for use in combating diseases.

4. Process for the preparation of compounds of the general formula (I)

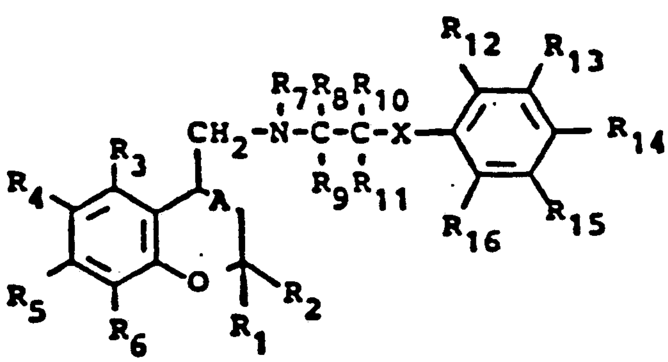

in which

-A- represents a single bond or a double bond, $R_1$ and $R_2$ are identical or different and represent hydrogen, $C_1$-$C_6$-alkyl, $C_5$-$C_7$-cycloalkyl, phenyl, optionally monosubstituted or disubstituted by $C_1$-$C_4$-alkyl, halogen and/or $C_1$-$C_4$-alkoxy, or $C_7$-$C_9$-aralkyl, the aryl radical of which is optionally monosubstituted or disubstituted by $C_1$-$C_4$-alkyl, halogen and/or $C_1$-$C_4$-alkoxy, or in which $R_1$ and $R_2$, together with the included C atom of the chroman ring, form a 4-membered to 7-membered carbocyclic ring;

$R_3$ to $R_6$ are identical or different and represent hydrogen, hydroxyl, halogen, $C_1$-$C_6$-alkyl, $C_5$-$C_7$-cycloalkyl, phenyl, optionally monosubstituted or disubstituted by $C_1$-$C_4$-alkyl, halogen and/or $C_1$-$C_4$-alkoxy, or $C_7$-$C_9$-aralkyl, the aryl radical of which is optionally monosubstituted or disubstituted by $C_1$-$C_4$-alkyl, halogen and/or $C_1$-$C_4$-alkoxy;

$R_7$, $R_8$, $R_9$, $R_{10}$ and $R_{11}$ are identical or different and represent hydrogen or $C_1$-$C_6$-alkyl,

X represents a single bond or methylene which is optionally monosubstituted or disubstituted by $C_1$-$C_4$-alkyl, or represents oxygen or $-NR_{17}$,

wherein

$R_{17}$ represents hydrogen or $C_1$-$C_4$-alkyl, or $R_{17}$ together with $R_7$ represents $C_2$-alkylene, wherein at least one and at most two of the radicals $R_{12}$, $R_{13}$, $R_{14}$, $R_{15}$ and $R_{16}$ are identical or different and represent nitro, cyano or one of the groups

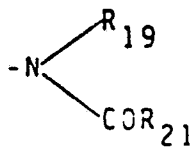 , 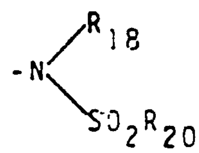 , 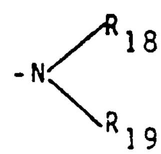 ,

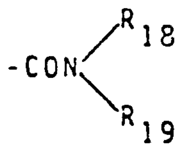 or 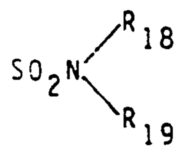

wherein

$R_{18}$ and $R_{19}$ are identical or different and each represent hydrogen or alkyl having 1 - 4 C atoms which is optionally monosubstituted to trisubstituted by halogen,

$R_{20}$ represents alkyl having 1-6 C atoms which is optionally monosubstituted to trisubstituted by halogen, and

$R_{21}$ represents hydrogen, alkyl, alkoxy or alkylamino, each having 1-4 C atoms per alkyl and alkoxy group, the alkyl radical being optionally monosubstituted to trisubstituted by halogen, or

wherein

$R_{18}$ together with $R_{19}$ or together with $R_{20}$ or together with $R_{21}$ can form a 5-membered to 6-membered heterocyclic ring which contains an alkyl bridge having 2, 3 or 4 carbon atoms and which optionally also contains an additional carbonyl group, whereas the remaining radicals $R_{12}$ to $R_{16}$ are identical or different and each represent hydrogen, hydroxyl, chlorine, fluorine, alkyl having 1 to 4 C atoms, alkoxy having 1 to 3 C atoms, aralkyloxy having up to 8 C atoms or trifluoromethyl,

and moreover

$R_{12}$ and $R_{13}$ or $R_{13}$ and $R_{14}$ together can represent an alkylenedioxy group having 1 or 2 C atoms or the group $-CH=CH-CH=CH-$,

and their pharmaceutically acceptable addition salts,

characterised in that

A) chroman-4-carbaldehydes of the formula (II)

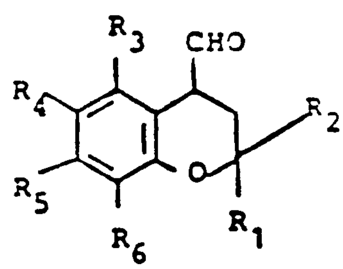

in which

$R_1$, $R_2$, $R_3$, $R_4$, $R_5$ and $R_6$ have the meaning given above,

are reacted with amines of the formula (III)

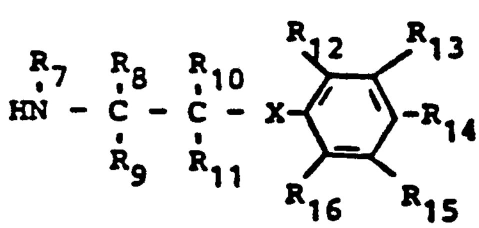

in which

$R_7$, $R_8$, $R_9$, $R_{10}$, $R_{11}$, $R_{12}$, $R_{13}$, $R_{14}$, $R_{15}$, $R_{16}$, $R_{17}$ and X have the meaning given above,

in the presence of reducing agents, or

B) amines of the formula (IV)

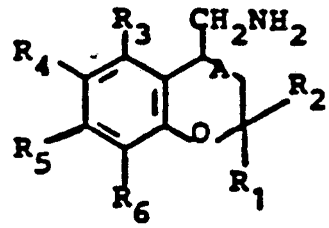

in which
A, $R_1$, $R_2$, $R_3$, $R_4$, $R_5$ and $R_6$ have the meaning given above, are reacted with carbonyl compounds of the formula (V)

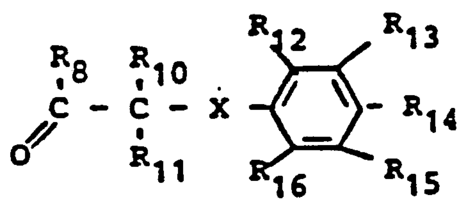

in which
$R_8$ to $R_{16}$ have the meaning given above, with the proviso that X does not represent $NR_{17}$, in the presence of reducing agents, or

C) compounds of the formula (VI)

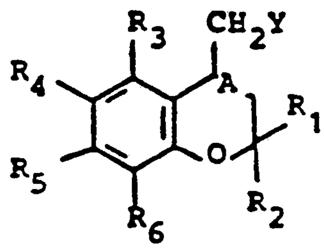

in which
A, $R_1$, $R_2$, $R_3$, $R_4$, $R_5$ and $R_6$ have the meaning given above, and
Y represents nucleophilically exchangeable groups, such as alkyl-substituted or aryl-substituted sulphonyloxy groups, bromine or chlorine,
are reacted with the amines of the formula (III) in the presence of an acid-binding agent.

5. Process according to Claim 4, characterised in that, in process variants A) and B), complex metal hydrides or catalytically activated hydrogen are used as reducing agents, and, in process variant C), metal hydroxides, carbonates or nitrogen bases are employed as acidbinding agents.

6. Process according to Claim 4, characterised in that the reactions are carried out at temperatures between -50 and +150°C.

7. Medicaments containing at least one compound of the general formula (I) according to Claim 1.

8. Process for the preparation of medicaments, characterised in that at least one compound of the general formula (I) according to Claim 1 is converted to a suitable form for administration, using inert auxiliaries and excipients.

9. Use of compounds of the general formula (I) according to Claim 1 for the preparation of medicaments for combating circulatory diseases.

**Revendications**

1. Benzopyrannes substitués de formule générale (I)

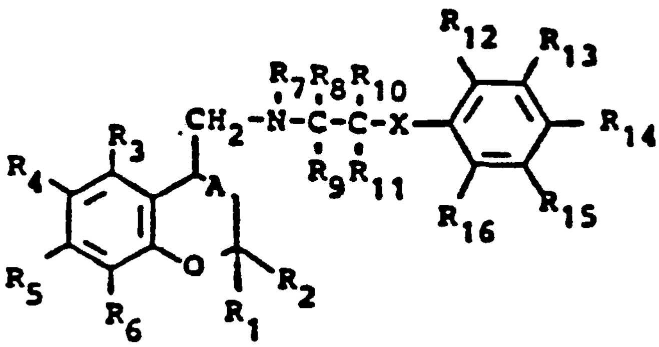

dans laquelle

-A- représente un liaison simple ou une double liaison,

$R_1$ et $R_2$, ayant des significations identiques ou différentes, représentent chacun l'hydrogène, un groupe alkyle en $C_1$-$C_6$, cycloalkyle en $C_5$-$C_7$, phényle éventuellement mono ou disubstitué par des groupes alkyles en $C_1$-$C_4$, des halogènes et/ou des groupes alcoxy en $C_1$-$C_4$, un groupe aralkyle en $C_7$-$C_9$ dont la partie aryle est éventuellement mono ou disubstituée par des groupes alkyles en $C_1$-$C_4$, des halogènes et/ou des groupes alcoxy en $C_1$-$C_4$, ou bien $R_1$ et $R_2$ forment ensemble et avec l'atome de carbone voisin du cycle chromane un noyau carbocyclique de 4 à 7 chaînons;

$R_3$ à $R_6$, ayant des significations identiques ou différentes, représentent chacun l'hydrogène, un groupe hydroxy, un halogène, un groupe alkyle en $C_1$-$C_6$, cycloalkyle en $C_5$-$C_7$, un groupe phényle éventuellement mono ou disubstitué par des groupes alkyles en $C_1$-$C_4$, des halogènes et/ou des groupes alcoxy en $C_1$-$C_4$, ou un groupe aralkyle en $C_7$-$C_9$ dont la partie aryle est éventuellement mono ou disubstituée par des groupes alkyles en $C_1$-$C_4$, des halogènes et/ou des groupes alcoxy en $C_1$-$C_4$;

$R_7$, $R_8$, $R_9$, $R_{10}$ et $R_{11}$, ayant des significations identiques ou différentes, représentent chacun l'hydrogène ou un groupe alkyle en $C_1$-$C_6$;

X représente une liaison simple, un groupe méthylène éventuellement mono ou disubstitué par des groupes alkyles en $C_1$-$C_4$, l'oxygène ou un groupe $-NR_{17}$, dans lequel $R_{17}$ représente l'hydrogène ou un groupe alkyle en $C_1$-$C_4$ ou bien encore $R_{17}$ forme, avec $R_7$, un groupe alkylène en $C_2$;

au moins un et au maximum deux des symboles

$R_{12}$, $R_{13}$, $R_{14}$, $R_{15}$ et $R_{16}$, ayant des significations identiques ou différentes, représentent des groupes nitro, cyano ou l'un des groupes

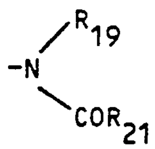 , 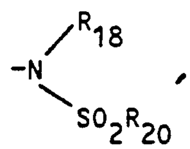 , 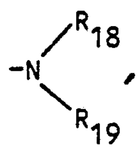 , 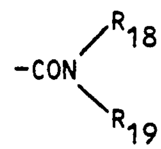 ou 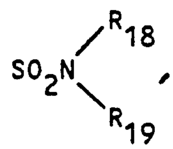 ,

dans lesquels

$R_{18}$ et $R_{19}$, ayant des significations identiques ou différentes, représentent chacun l'hydrogène ou un groupe alkyle en $C_1$-$C_4$ éventuellement mono à trisubstitué par des halogènes,

$R_{20}$ représente un groupe alkyle en $C_1$-$C_6$ éventuellement mono à trisubstitué par des halogènes et

$R_{21}$ représente l'hydrogène, un groupe alkyle, alcoxy ou alkylamino contenant chacun 1 à 4 atomes de carbone par partie alkyle et alcoxy, la partie alkyle pouvant le cas échéant être mono à trisubstituée par des halogènes,

ou bien

$R_{18}$ forme, avec $R_{19}$ ou avec $R_{20}$ ou avec $R_{21}$ un noyau hétérocyclique de 5 à 6 chaînons qui contient un pont alkyle à 2, 3 ou 4 atomes de carbone et le cas échéant en outre un groupe carbonyle,

cependant que les autres symboles $R_{12}$ à $R_{16}$, ayant des significations identiques ou différentes, représentent chacun l'hydrogène, un groupe hydroxy, le chlore, le fluor, un groupe alkyle en $C_1$-$C_4$, alcoxy en $C_1$-$C_3$, aralkyloxy contenant jusqu'à 8 atomes de carbone ou trifluorométhyle,

et en outre,

$R_{12}$ et $R_{13}$ ou $R_{13}$ et $R_{14}$ peuvent former ensemble un groupe alkylène-dioxy à 1 ou 2 atomes de carbone ou le groupe $-CH=CH-CH=CH-$ ;

et leurs sels formés par addition et acceptables pour l'usage pharmaceutique.

2. Composés de formule générale (I) selon la revendication 1, dans lesquels:

$R_1$ et $R_2$, ayant des significations identiques ou différentes, représentent chacun l'hydrogène, un groupe alkyle en $C_1$-$C_4$ ou forment ensemble et avec l'atome de carbone voisin, un noyau carbocyclique en $C_5$ ou $C_6$,

$R_3$ à $R_6$, ayant des significations identiques ou différentes, représentent chacun l'hydrogène, un groupe hydroxy, alkyle en $C_1$-$C_4$, alcoxy en $C_1$-$C_4$ ou le chlore,

$R_7$ à $R_{11}$, ayant des significations identiques ou différentes, représentent chacun l'hydrogène ou un groupe alkyle en $C_1$-$C_4$,

X représente une liaison simple, l'oxygène, un groupe méthylène ou $-NR_{17}$, dans lequel $R_{17}$ représente l'hydrogène ou un groupe alkyle en $C_1$-$C_3$, ou bien $R_{17}$ forme avec $R_7$ un groupe éthylène qui ferme le cycle, et

$R_{12}$ à $R_{16}$, ayant des significations identiques ou différentes, représentent chacun l'hydrogène, le chlore, un groupe cyclohexyle, alkyle en $C_1$-$C_4$, alcoxy en $C_1$-$C_3$, aralcoxy en $C_1$-$C_8$, trifluorométhyle, ou bien $R_{12}$ et $R_{13}$ ou $R_{13}$ et $R_{14}$ forment le cas échéant, pour chaque paire, un groupe méthylène-dioxy ou un groupe $-CH=CH-CH=CH-$,

au moins un et au maximum deux symboles du groupe $R_{12}$ à $R_{16}$, ayant des significations identiques ou différentes, représentent chacun un groupe nitro, cyano, le groupe

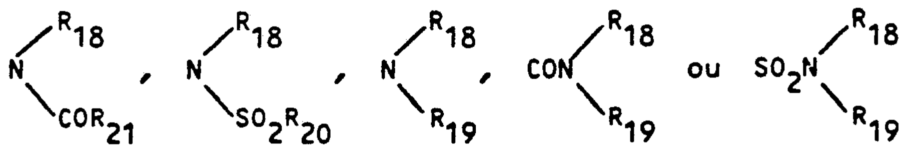

dans lequel

$R_{18}$ et $R_{19}$, ayant des significations identiques ou différentes, représentent chacun l'hydrogène ou un groupe alkyle en $C_1$-$C_4$ éventuellement substitué par des halogènes et $R_{20}$ représente un groupe alkyle en $C_1$-$C_4$ éventuellement substitué par le fluor ou le chlore, $R_{21}$ représente l'hydrogène, un groupe alkyle en $C_1$-$C_4$ éventuellement substitué par le fluor ou le chlore, un groupe alcoxy ou alkylamino contenant chacun 1 ou 2 atomes de carbone.

3. Composés de formule générale (I) selon la revendication 1 pour l'utilisation dans le traitement de maladies.

4. Procédé de préparation des composés de formule générale (I)

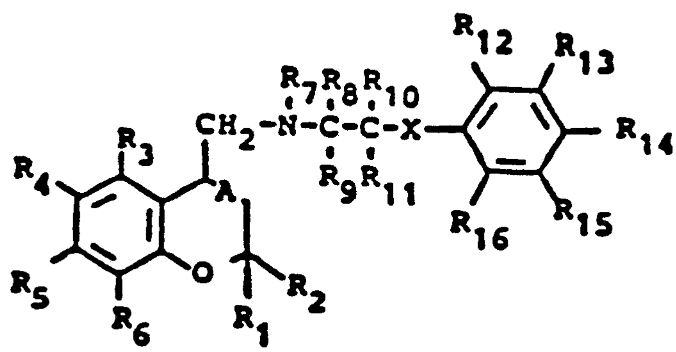

dans laquelle

-A- représente un liaison simple ou une double liaison,

$R_1$ et $R_2$, ayant des significations identiques ou différentes, représentent chacun l'hydrogène, un groupe alkyle en $C_1$-$C_6$, cycloalkyle en $C_5$-$C_7$, phényle éventuellement mono ou disubstitué par des alkyles en $C_1$-$C_4$, des halogènes et/ou des groupes alcoxy en $C_1$-$C_4$, un groupe aralkyle en $C_7$-$C_9$ dont la partie aryle est éventuellement mono ou disubstituée par des groupes alkyles en $C_1$-$C_4$, des halogènes et/ou des groupes alcoxy en $C_1$-$C_4$, ou bien $R_1$ et $R_2$ forment ensemble et avec l'atome de carbone voisin du cycle chromane, un noyau carbocyclique de 4 à 7 chaînons;

$R_3$ à $R_6$, ayant des significations identiques ou différentes, représentent chacun l'hydrogène, un groupe hydroxy, un halogène, un groupe alkyle en $C_1$-$C_6$, cycloalkyle en $C_5$-$C_7$, un groupe phényle éventuellement mono ou disubstitué par des groupes alkyles en $C_1$-$C_4$, des halogènes et/ou des groupes alcoxy en $C_1$-$C_4$, ou un groupe aralkyle en $C_7$-$C_9$ dont la partie aryle est éventuellement mono ou disubstituée par des groupes alkyles en $C_1$-$C_4$, des halogènes et/ou des groupes alcoxy en $C_1$-$C_4$;

$R_7$, $R_8$, $R_9$, $R_{10}$ et $R_{11}$, ayant des significations identiques ou différentes, représentent chacun l'hydrogène ou un groupe alkyle en $C_1$-$C_6$;

X représente une liaison simple, un groupe méthylène éventuellement mono ou disubstitué par des groupes alkyles en $C_1$-$C_4$, l'oxygène ou un groupe $-NR_{17}$, dans lequel $R_{17}$ représente l'hydrogène ou un groupe alkyle en $C_1$-$C_4$ ou bien encore $R_{17}$ forme, avec $R_7$, un groupe alkylène en $C_2$;

au moins un et au maximum deux des symboles

$R_{12}$, $R_{13}$, $R_{14}$, $R_{15}$ et $R_{16}$, ayant des significations identiques ou différentes, représentent des groupes nitro, cyano ou l'un des groupes

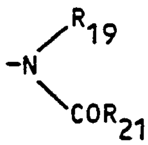 , 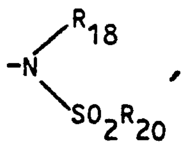 , 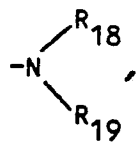 , 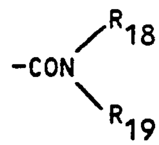 ou 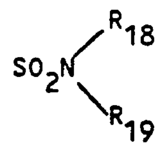

dans lesquels

$R_{18}$ et $R_{19}$, ayant des significations identiques ou différentes, représentent chacun l'hydrogène ou un groupe alkyle en $C_1$-$C_4$ éventuellement mono à trisubstitué par des halogènes,

$R_{20}$ représente un groupe alkyle en $C_1$-$C_6$ éventuellement mono à trisubstitué par des halogènes et

$R_{21}$ représente l'hydrogène, un groupe alkyle, alcoxy ou alkylamino contenant chacun 1 à 4 atomes de carbone par partie alkyle et alcoxy, la partie alkyle pouvant le cas échéant être mono à trisubstituée par des halogènes,

ou bien

$R_{18}$ forme, avec $R_{19}$ ou avec $R_{20}$ ou avec $R_{21}$ un noyau hétérocyclique de 5 à 6 chaînons qui contient un pont alkyle à 2, 3 ou 4 atomes de carbone et le cas échéant en outre un groupe carbonyle,

cependant que les autres des symboles $R_{12}$ à $R_{16}$, ayant des significations identiques ou différentes, représentent chacun l'hydrogène, un groupe hydroxy, le chlore, le fluor, un groupe alkyle en $C_1$-$C_4$, alcoxy en $C_1$-$C_3$, aralkyloxy contenant jusqu'à 8 atomes de carbone ou trifluorométhyle,

et en outre,

$R_{12}$ et $R_{13}$ ou $R_{13}$ et $R_{14}$ peuvent former ensemble un groupe alkylène-dioxy en $C_1$ ou $C_2$ ou le groupe -CH=CH-CH=CH-;

et leurs sels formés par addition et acceptables pour l'usage pharmaceutique,

caractérisé en ce que:

A) On fait réagir des chromane-4-carbaldéhydes de formule

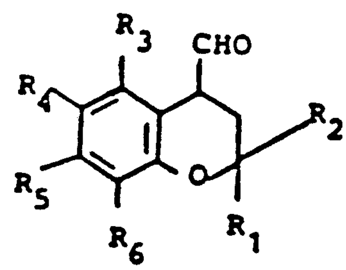

dans laquelle

$R_1$, $R_2$, $R_3$, $R_4$, $R_5$ et $R_6$ ont les significations indiquées ci-dessus,

avec des amines de formule

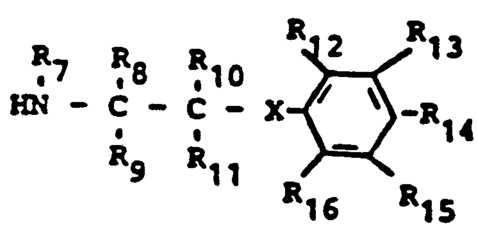

dans laquelle

$R_7$, $R_8$, $R_9$, $R_{10}$, $R_{11}$, $R_{12}$, $R_{13}$, $R_{14}$, $R_{15}$, $R_{16}$, $R_{17}$ et X ont les significations indiquées ci-dessus,

en présence d'agents réducteurs, ou bien

B) On fait réagir des amines de formule (IV)

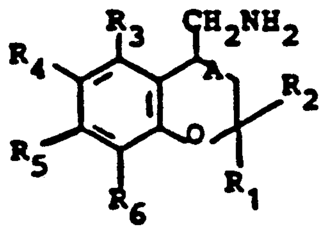

dans laquelle
A, $R_1$, $R_2$, $R_3$, $R_4$, $R_5$ et $R_6$ ont les significations indiquées ci-dessus,
avec des composés carbonylés de formule (V)

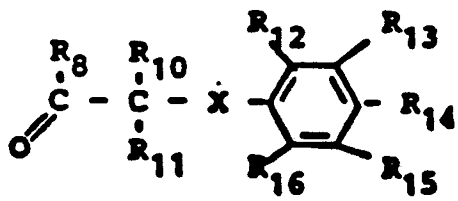

dans laquelle
$R_8$ à $R_{16}$ ont les significations indiquées ci-dessus, sous réserve que X ne peut représenter $NR_{17}$, en présence d'agents réducteurs, ou bien

C) On fait réagir des composés de formule (VI)

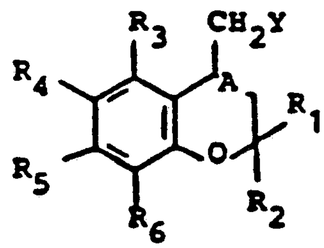

dans laquelle
A, $R_1$, $R_2$, $R_3$, $R_4$, $R_5$ et $R_6$ ont les significations indiquées ci-dessus et
Y représente un groupe nucléophile échangeable tel qu'un groupe sulfonyloxy à substituant alkyle ou aryle, le brome ou le chlore, avec les amines de formule (III) en présence d'un capteur d'acide.

5. Procédé selon la revendication 4, caractérisé en ce que, dans les variantes opératoires A) et B), on utilise en tant qu'agents réducteurs des hydrures métalliques complexes ou de l'hydrogène activé par un catalyseur, et dans la variante opératoire C), on utilise en tant que capteur d'acide un hydroxyde ou carbonate métallique ou une base azotée.

6. Procédé selon la revendication 4, caractérisé en ce que les réactions sont effectuées à des températures allant de - 50 à + 150°C.

7. Médicament contenant au moins un composé de formule générale (I) selon la revendication 1.

8. Procédé de préparation de médicaments, caractérisé en ce que l'on met au moins un composé de formule générale (I) selon la revendication 1 sous une forme d'administration appropriée par utilisation de produits auxiliaires et véhicules inertes.

9. Utilisation des composés de formule générale (I) selon la revendication 1 pour la préparation de médicaments pour le traitement des maladies circulatoires.